# EUROPEAN PATENT SPECIFICATION

(11) **EP 1 206 563 B1**
(45) Date of publication and mention of the grant of the patent: **27.01.2010**
(21) Application number: 00957305.6
(22) Date of filing: 07.08.2000
(51) Int. Cl.: C12N 15/864, C12N 5/10, C12N 15/19, C07K 14/52, A61K 35/12

(54) **GENETICALLY ALTERED DENDRITIC CELLS TRANSDUCED WITH ADENO-ASSOCIATED VIRUS (AAV), METHODS OF PRODUCING SAID CELLS AND USES THEREOF**
GENETISCH VERÄNDERTE DENDRITISCHE ZELLEN, DIE MIT ADENO-ASSOZIIERTEM VIRUS TRANSDUZIERT WURDEN, VERFAHREN ZUR HERSTELLUNG DIESER ZELLEN UND IHRE VERWENDUNGEN
CELLULES DENDRITIQUES GENETIQUEMENT MODIFIEES TRANSDUCTEES AVEC UN VIRUS ADENO-ASSOCIE (AAV), PROCEDES DE FABRICATION ET D'UTILISATION DE CES CELLULES

(30) Priority: 05.08.1999 US 147263 P
(43) Date of publication of application: 22.05.2002
(73) Proprietor: THE BOARD OF TRUSTEES OF THE UNIVERSITY OF ARKANSAS, Little Rock, AR 72207-3608 (US)
(72) Inventor: HERMONAT, Paul, L., Little Rock, AR 72227 (US); SANTIN, Alessandro, D., Little Rock, AR 72207 (US); LIU, Yong, Little Rock, AR 72205 (US); MANE, Michael, Little Rock, AR 72217 (US); PARHAM, Groesbeck, P., Little Rock, AR 72211 (US); CHIRIVA-INTERNATI, Maurizio, Birmingham, AL 35242 (US)
(74) Representative: Vossius & Partner
(86) International application number: PCT/US2000/021410
(87) International publication number: WO 2001/011067

(56) References cited:
- WO-A-97/03703
- WO-A-97/29183
- WO-A-98/24479
- KROUSE R. S. ET AL.: "rAAV Vector transduction of dendritic cells (DC) for development of tumor specific vaccines." BLOOD, vol. 92, no. 10 SUPPL. 1 PART 1-2, 15 November 1998 (1998-11-15), pages 146A-147A, XP000978926 40th Annual Meeting of the American Society of Hematology; Miami Beach, Florida, USA; December 4-8, 1998 ISSN: 0006-4971

## Description

### BACKGROUND OF THE INVENTION

This application claims priority to U.S. Serial No. 60/147,263 filed on August 5, 1999.

This present invention is directed to a method for preparing genetically altered dendritic cells (DC) using an adeno-associated virus (AAV) vector as the vehicle for introducing heterologous gene(s) into the DCs. The present invention also is directed to the DCs produced by this method. The AAV vector is operably linked to at least one heterologous gene, which preferably encodes a cytokine or an antigen associated with a disease or combinations of one or more of each of these genes. The present invention also is directed to the use of the patient's antigen presenting DCs that have been genetically altered to contain at least one heterologous gene, such as a disease specific antigen, that is preferably stably integrated into the DCs' genome in the preparation of a pharmaceutical composition for an immuno-stimulatory treatment for a disease, particularly cancer, such as cervical cancer, breast cancer and muliple myeloma, or an infectious disease, such as Hepatitis B or C virus, AIDS, or warts. Any antigen that is a marker for a specific disease, such as infectious diseases or a tumor specific marker, can be delivered to a DC via AAV vector delivery, according to the present invention. These DCs express the gene encoding the antigen, process the antigen and present this antigen as an antigenic epitope for T-cell priming. But even genetically altered DC that carries a heterolgous gene encoding a cytokine is useful for treating cancers or other diseases that would benefit from cytokines treatment because the expression of cytokines by the DCs stimulates the basal level of tumor cell recognition and enhanced the immune response. Additionally, these genetically altered DCs are useful to stimulate the patient's own T-cells *in vivo* and/or these genetically altered DCs are useful to stimulate T-cells *in vitro,* and then these primed T-cells are to be administered to the patient to stimulate the patient's immune system via adoptive immunotherapy.

DCs, although rare, are potent and the most effective antigen presenting cells (APC), which are able to initiate primary immune response to antigens by naive T cells.(1) Several research groups (2,3) have developed tissue culture protocols for generating large numbers of DCs *in vitro* from peripheral blood in the presence of GM-CSF and IL-4. Recent studies (64,100) have established the key role played by DCs in the immune system and provide a rationale for using DCs as natural adjuvants for human immunotherapy (4). Thus, the ability to culture DCs from peripheral blood allows DCs to be used to manipulate the immune response of autologous human T cells (4). This manipulation offers great promise as an immunotherapeutic adjunct to current cancer and infectious disease therapies.

There have been recent reports showing the possibility of inducing specific T cell responses against tumor challenge *(in vivo*), or tumor cells *(in vitro),* using enriched DCs which had been pulsed or incubated with tumor fragments, antigen peptides, defined tumor antigens, or with antigen genes.(4-33) In addition to potential degradation problems for all proteins and peptides that are used to pulse DCs, bacterially produced antigen proteins lack mammalian post-translational modifications, and potentially may lack antigenic epitopes. ' There are several potential ways to treat or pulse cells with proteins. Such techniques .. ' include lipofection, electroporation, tat-tagging, microinjection; and scrape loading.(34-41) However, the disadvantages of these various methods are that most of these proteins have a very short half-life and are degraded. Likewise, it is known that the cytokines, granulocyte macrophage-colony stimulating factor (GM-CSF) and interleukin-4 (IL-4), are needed for the generation of dendritic cells. These cytokines are incubated with DCs but these proteins also have limited half-lives as active proteins(42) and can be degraded as well.

The helper dependent parvovirus adeno-associated virus (AAV) is able to patently infect cells via chromosomal integration. Early studies on AAV latency from 1970 to 1986 demonstrate that 15-30% of immortalized cells could be latently infected with wild type AAV and that the AAV genome was-chromosomally integrated.(74-76) Upon mapping of AAV's genes and their functions (86,87), recombinant AAV virus vectors were demonstrate to have a similar ability in immortalized tissue culture cells (43,88), and in 1988, recombinant AAV transduction of primary hematopoietic stem cells was achieved (44). More recently, a region of human chromosome 19 was found to be the preferred site of wild type AAV integration.(89-91) Recombinant AAV stably transduces cells by chromosomal integration (43,51,65) and transduced cells are not usually a significant target of the immune system.(79-81) A number of laboratories have since confirmed and extended this data by demonstrating the utility of AAV based vectors.(45,49,67,81, 92-96) In the last few years, the likely initiation of the deciphering of the mechanism of wild type AAV integration has begun with the finding that a complex between chromosome 19 DNA and AAV terminal repeat DNA was formed in the presence of the AAV Rep78/68 protein (100), the same protein which is required for AAV DNA replication. (86,87)

Thus, AAV has been shown to be a versatile virus vector, being able to transduce a wide variety of cell types, including an effective gene delivery vector for both immortalized tissue culture cells, as well as primary hematopoietic cells. (45,49,67,81. 92-96). AAV is an efficient delivery vector that generates minimal intrinsic immunogenicity. Other viruses, including retroviruses, adenoviruses, and pox viruses, have previously been shown to transduce DCs (9,11,24,29), but no one has been able to show the transduction of DCs with AAV. This may be so because AAV prefers transducing host cells that are actively dividing rather than quiescent cells, such as DCs that do not divide. But AAV does not possess the drawbacks of these other viruses that includes their large size and that they carry extra genes into the transduced DCs. Thus, these larger viruses are not particularly useful for an immunotherapy treatment that focuses on obtaining an immune response to a particular antigen because these additional proteins elicit immune responses and do not allow the patient's immune system to focus on the immune response to the particular antigen. AAV is smaller in size and presents less complicated epitopes to the patient then retroviruses or adenoviruses. Additionally and very importantly, the retroviruses and retroviral vectors have been banned for use in gene therapy by the Food and Drug Administration. For all of these reasons, a more suitable choice of a vector for transducing DCs, such as an AAV vector, is needed.

Human papillomavirus (HPV) infection represents the most important risk factor for developing cervical cancer. Although there are over 20 oncogenic HPV genotypes, HPV-16 and -18 are the most prevalent in cervical cancers regardless of geographic location. The E6 and E7 transforming oncoproteins of these two viruses are detected in the vast majority of HPV-positive cancer biopies and almost all HPV-containing cell lines and they play a crucial role in both transformation and maintenance of the malignant phenotype. Therefore, E6 and E7 are ideal candidates as potential tumor specific targets for cervical cancer immunotherapy. Because of the unique viral antigens/viral oncoproteins HPV (E6/E7), which are always present in cervical cancer (>90%) but are not usually present in normal tissues, cervical cancer is an ideal cancer for testing the promise of immunotherapy according to the present invention. Furthermore, cervical cancer is a very important cancer world-wide because it ranks just behind breast cancer in its prevalence in world statistics but, in fact, ranks higher than breast cancer in the developing world.

Several lines of evidence suggest that cell-mediated immune responses are important in controlling both HPV infections and HPV-associated neoplasms. First, there is an increased incidence of associated genital cancer in immunosuppressed patients while only a minority of genital HPV infections result in the development of cancer in otherwise healthy individuals. Second, infiltrating CD4+ (T helper cells) and CD8+ (cytotoxic/suppressor T cells) T cells have been observed in spontaneously regressing warts. Third, studies on animals have demonstrated that immunized animals are protected from papillomavirus infections and from transplanted tumor cells expressing HPV E6/E7 viral proteins.

The immune response may be limited or inhibited in cervical cancer patients. Despite the above observations, to date, only a few reports have demonstrated the generation of HPV E6 and E7 specific CTLs in cervical cancer patients, suggesting that such CTL precursors may be present at low levels. However, HPV naturally infected targets (e.g., keratinocytes) are known to express low levels of viral proteins and MHC (major histocompatibility complex) restriction elements as well as lack of costimulation molecules crucial for naive T cell priming. Therefore, presentation of viral antigens by these non-professional APCs may in part explain the capacity of HPV to evade the host immune system. Consistent with this hypothesis, hyporesponsiveness or tolerance have been previously reported following presentation of "non-self" antigens by keratinocytes.

Another type of cancer that can benefit from the immuno-stimulatory treatment of the present invention is breast cancer because specific antigens have been identified that are associated with breast carcinomas. Breast cancer vies with lung cancer as one of the most prevalent cancers. There have been many studies demonstrating the defective function of T lymphocytes in cancerous hosts (101). This also has been shown to be true in breast cancer. (102) In this study, DCs and T cells, taken from breast cancer patients, showed a decrease in proliferative T cell response to Concanavalin A (ConA), tetanus toxoid, or allogeneic T cells, when compared to controls in a mixed lymphocyte reaction. Further, the degree of defectiveness was dependent upon the stage of breast cancer. In stage 4 patients, the most invasive, the defect in proliferation was as much as 85%. The defect was specifically localized to the DCs and not the T cells, by combining various cell populations. Finally, it was found the if DCs were generated from peripheral blood monocytes from these stage 4 breast cancer patients, no defect was found in their ability to stimulate T cell response. Thus, there was no intrinsic defectiveness to the patients DCs, just those isolated as mature DCs. Clearly, the effectiveness of these mature DCs were down regulated *in vivo.* The present invention avoids this by generating DCs from peripheral blood.

The methods, AAV vectors and genetically altered DCs of the present invention are useful to treat breast cancer by utilizing identified breast cancer associated antigens. A number of breast cancer antigens have been identified, but many are expressed only in a limited number of breast cancers. It is important to select a breast cancer antigen for use in the present invention, that is expressed in a large number of breast tumors. Three breast cancer antigens, BA46, Her-2/neu, and CD24, are among such antigens that are useful in the present invention, as a result of their broad expression in a great number of breast tumors.

BA46 (also called Lactadherin) is a membrane-associated glycoprotein which is almost universally expressed in breast carcinomas. This protein is a part of milk and has been referred to as "milk fat globule membrane." (103) But this protein is also expressed in most breast cancer cells, being found expressed in 7 of 7 of such cell lines (104). Furthermore, the protein/antigen is expressed on the cell surface (105-107). Antibodies against this protein also are available (108-110). The antigen has been cloned and its size of 46 kDa, translating to a size of about 1 kilobase of DNA, can easily fit inside an AAV vector.

A large number of studies has established that Her-2/neu is an important oncogene involved in breast cancer. ErbB2 or Her-2/neu was originally isolated as a defective retroviral viral oncogene in the chicken erythroblastosis virus (111). A normal cellular homologue (proto-oncogene) to the viral oncogene was soon discovered (112). Several studies have demonstrated that Her-2/neu protein is over expressed in 40-50% of breast cancer tissues compared to adjacent normal tissue (113,114). The over expression of this protein appears to be linked with the preferential amplification of this gene in these breast cancers (115,116). This has been recognized by others and, thus, Her-2/neu (erbB2) has been thought to be a potential target for immunotherapy as early as 1990 (117).

CD24 is a cell surface protein and appears to be a ligand for P-selectin and this interaction is important for leukocyte-endothelium interaction (118-120). It is a small protein of only 27 amino-acids and undergoes extensive post translational modification, being highly glycosylated and bound to the membrane via a phosphatidylinositol anchor (120). CD24 appears to be expressed on several cancers, including small cell carcinoma, neuroblastoma, rhabdomyosarcoma and renal cell carcinoma. (121,122) Also, CD24 appears to be highly expressed in ductal breast cancers, being over expressed in 31 of 31 such tissues by immunohistochemistry (123), but a disadvantage is that CD24 also is expressed on some B cells during early stages of B cell development (124).

The antigens, BA46 and CD24 have not been used as immunotherapeutic targets. However, Her-2/neu has been used as an antigenic target by a number of studies for breast and pancreatic cancer (125-129). Three studies have used Her-2/neu derived oligopeptides transfected into DCs to elicit an immunoresponse and these treatments have generated some success. (126,127,129). Another technique has been the use of a fibroblast cell line which overexpressed Her-2/neu as the immunogen that was partially successful. (127)

Although all of these studies showed some level of successful immunostimulation, these techniques may have flaws. The use of oncogene altered fibroblasts and oncogenic plasmids will likely not be used clinically as they are potentially dangerous. Additionally, the use of small oligopeptides, representing a single epitope, may not induce the maximum stimulation of immune response. The present invention overcomes this deficiency by transferring the complete antigen gene into the DC, itself, and uses these transduced DCs as a technique to obtain the maximum stimulation of the immune response on a continuous basis.

The present invention shows for the first time that AAV transduces and chromosomally integrate into cells of the dendritic lineage. The present method utilizes AAV to transduce DCs with heterologous genes that are integrated into the genome of the DCs. The present method provides improvements in DC production and function resulting in a superior cytotoxic CD8+ T cell response and CD4+ helper T cell proliferation *in vitro* and *in vivo.* The DCs produced by the present invention are useful in studying DC function and their role in the immune system as APCs. Additionally, these genetically altered DCs are useful as an effective immuno-therapeutic regime for cancers and infectious diseases that involves the manipulation of these APCs. Immunizations with DCs transduced with tumor antigens or antigens associated with infectious diseases, or T-cells stimuated with these DCs provide effective uses of these DCs in the preparation of a pharmaceutical composition for inducing antitumor or anti-infectious disease immunity in a patient.

The present invention also provides a more effective technique for treating DCs with proteins, such as antigens and/or cytokines, by transducing the genes encoding these proteins into the DCs via AAV delivery. The integration of these genes into the genome of the DCs, with subsequent expression of these genes, provides an improvement over the incubation of DCs with these proteins. By delivering the tumor antigen gene directly into the DCs, a stable and continuous production of the tumor antigen is accomplished. (8,9,11.12.18,24, 26, 29,30) Delivery of the entire gene has other advantages relevant to the induced immune response. Many DC-pulsing protocols use antigen-derived oligopeptides, which represent a very limited number of epitopes compared to the entire protein. This poses a major constraint to therapeutic potential as the T-cell immune response is limited to those epitopes presented by host HLA molecules. In constrast, the use of autologous DCs treated with a whole gene to produce the full length protein allows for the likely processing and presentation of many epitopes in association with host HLA molecules. Furthermore, HLA/MHC class. I and classII molecules degrade, are lost, or reabsorbed (34,98,99). There is evidence that the half-life of class I molecules correlates with the efficiency of the cytotoxic T cell response generated against a particular antigen. (98,99) Since the half-life of class I molecules can range from 2 to 22 hours, the loss of the surface molecule carrying the epitope must be added to the loss of the antigen protein itself when calculating the loss of antigen presentation. However, the present method provides for the continuous production of the antigen and/or cytokine and overcomes any immune deficits resulting from the continual recycling of class I and II molecules and loss of sensitizing epitopes. Further, the method of the present invention introduces more epitopes than by the above methods of oligopeptide introduction or pulsing of DCs. Additionally, it is expected that AAV delivery of an antigen results in higher class I antigen epitope presentation. Further, the present method provides epitopes that are generated and presented endogenously, which is intended to be more representative than epitopes of proteins that are produced in bacteria and delivered to the DCs by way of incubation or pulsing.

The present invention is the first reported successful DC transduction by AAV with the stable integration of the transduced gene into the DC's genome. Although an abstract from a grant application, grant number 5R01CA75186-02, to the National Cancer Institute describes transforming DCs with HPV E7 using AAV, this disclosure is merely a proposed experiment in the grant request. The abstract states that the researcher will test the induction of HLA-restricted, antigen-specific CTL following introduction of the HPV E7 transforming gene into DCs by AAV vector transduction. Applicants contend that this grant application is not an enabling disclosure and provides no evidence that the described prophetic research proposal was successful.

The present invention utilizes AAV to transduce at least one heterologous gene into DCs: The resulting AAV transduced DCs, produced according to the present invention, display many of the markers characteristic of mature DCs, supporting the usefulness of the present invention as outlined recently. (139) The present invention discloses that AAV transduces and stably integrates into the genome cells of the dendritic lineage, and thus represents a step forward in providing an excellent APC for studying DC function and its role in the immune system. The present invention also provides a genetically altered DC for use in immuno-therapy containing genes encoding a specific antigen associated with a disease and/or a cytokine or combinations of one or more of each, that provider improvements in DC production and function resulting in a superior cytotoxic CD8 + T cell response and CD4+ helper T cell proliferation.

### SUMMARY OF THE INVENTION

The present invention is based upon the successful transduction of DCs by an AAV vector containing a heterologous gene to obtain genetically altered DCs expressing the heterologous gene in the DCs.

In one embodiment, a method for preparing genetically altered DCs is provided This method comprises: (a) obtaining DC precursors from a human blood sample; (b) preparing adherent dendrirtic cell precursors from said dendritic cell precursors; and (c) transducing the DC precursors with at least one AAV vector comprising at least one heterologous gene to obtain genetically altered DCs expressing said heterolgous gene.

In a further embodiment, the present invention is directed to an adeno-associated virus (AAV) vector comprising at least a portion of the AAV genome and at least one heterologous gene selected from the group consisting of a gene encoding an antigen, a gene encoding a cytokine and combinations of one or more genes thereof.

In an additional embodiment, the genetically altered DCs produced by the methods of the present invention also are provided. These altered DCs are able to express one or more of the heterologous gene that have been transduced into the DCs.

In a further embodiment, an AAV vector is provided that comprises at least a portion of the AAV genome and at least one heterologous gene selected from the group consisting of a genre encoding an antigen, a gene encoding a cytokine and combinations of one or more genes thereof, and also provided is a DC containing this vector.

In another embodiment, the present invention is directed to a use of genetically altered DCs comprising at least one AAV vector comprising at least one heterologous gene selected from the group consisting of a gene encoding an antigen associated with said disease, a gene encoding a cytokine and combinations of one or more genes thereof in the preparation of a pharmaceutical composition for stimulating the immune system of a patient afflicted with a disease, whereby the heterologous gene is expressed in the DCs and the patient's immune system is stimulated by the stimulation of the patient's T cell response.

Also described herein is a method for stimulating the immune system of a patients afflicted with a disease comprises administering to the patient stimulated T cells, wherein the T-cells were stimulated by genetically altered DCs comprising at least one AAV vector comprising at least one heterologous gene selected from the group consisting of a gene encoding an antigen associated with the disease, a gene encoding a cytokine and combinations of one or more genes thereof, whereby the heterologous gene is expressed in the DCs and the patient's immune system is stimulated by the patient's T-cell response.

In an additional embodiment, the methods of the present invention are useful for generating DCs that can be manipulated, studied, utilized in *in vitro* experiments and used in the preparation of a pharmaceutical composition for the treatment of patients.

In a further embodiment, the methods of the present invention are useful in generating DCs for use in the preparation of a pharmaceutical composition for the general stimulation of the immune response in animals and humans.

In another embodiment, the invention is directed to a pharmaceutical composition comprising genetically altered DCs comprising the AAV vector comprising at least one heterologous gene selected from the

### BRIEF DESCRIPTION OF THE DRAWINGS

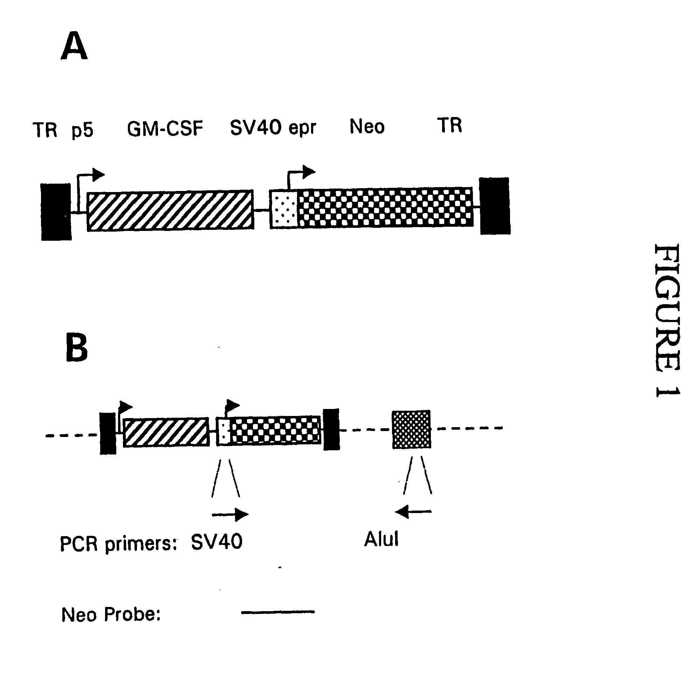

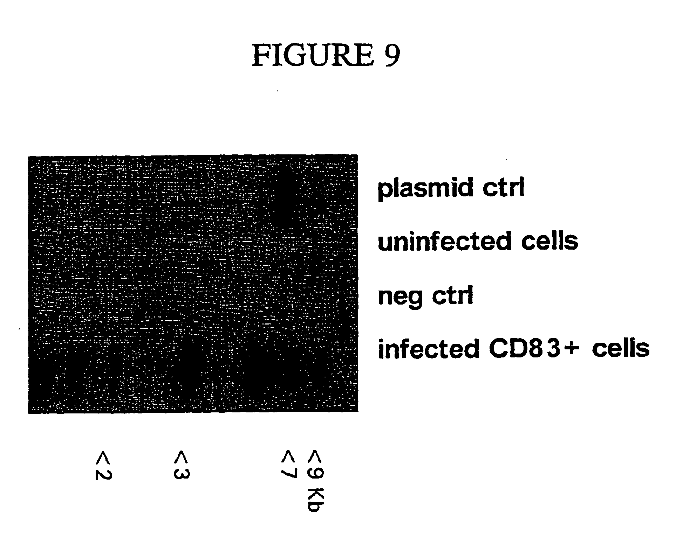

Figure 1 shows the map of the d16-95/GM-CSF^{p5}/Neo^{5v40} virus vector and primer/probe design for PCR/Southern blot analysis of integrated provirus. Figure 1A shows a structural map of the dl6-95/GM-CSF^{p5}/Neo^{SV40} virus with the names of the components at the top. TR (black box) refers to the AAV terminal repeats. p5 (bent arrow) refers to the AAV p5 promoter. The cross hatched box is the GM-CSF open reading frame. SV40 epr (stipled box/bent arrow) refers to the SV 40 early enhancer/promoter. The checkered box is the Neo open reading frame. Figure 1B shows the location of the PCR primers and the probe by which chromosomal integration was demonstrated in Figure 9.

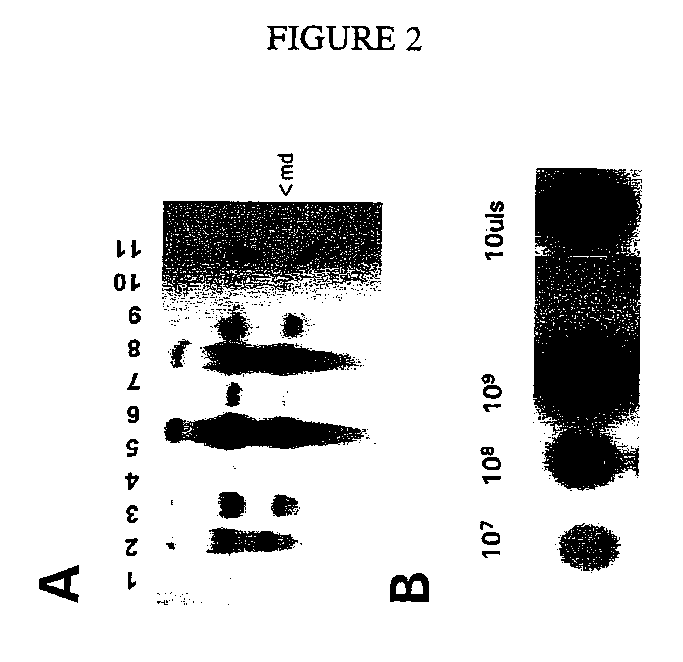

Figure 2 shows the production and characterization of AAV/GM-CSF/Neo virus stock. Figure 2A shows a Southern blot of 11 putative producer cell lines as described in the Materials and Methods. The blot was probed with ³²P-Neo DNA. Note that 293-AGN clones 5 and 7 produced the highest levels of AAV/GM-CSF/Neo DNA replication. Figure 2B shows a titering dot blot hybridization of the AAV/GM-CSF/Neo virus stock generated from clone 293-AGN-5. On the left are three spots in which 10⁷, 10⁸, and 10⁹ AAV/GM- . CSF/Neo plasmid genomes have been applied as titering standards. On the right the DNA from 10 uls of AAV/GM-CSF/Neo has been applied. This DNA was isolated as described in the Materials and Methods section. Note that the virus stock gave a signal similar to the 10⁹ standard, giving a titer of 10¹¹ encapsidated genomes for that virus stock.

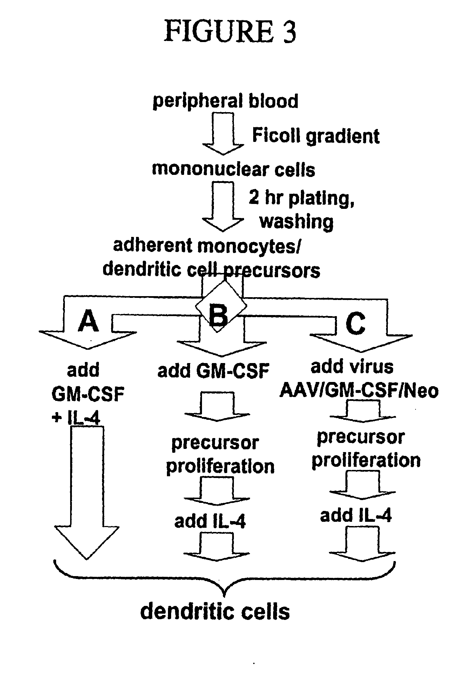

Figure 3 shows the overview of culture techniques for generating DCs. Method A shows the traditional technique of Sallusto and Lanzavecchia (1994) and Romani *et al.* (1994) for generating primary cell cultures enriched for DCs (2,3). Method B shows an alteration of this technique which allows for a period of precursor cell proliferation. In this alteration the cells are treated sequentially, first with GM-CSF, then with IL-4. This technique also results in DCs, but the resulting population is slightly less pure. Method C shows the primary technique used in the present invention which is an alteration of B in which exogenous GM-CSF addition is replaced by infection with the AAV/GM-CSF/Neo virus.

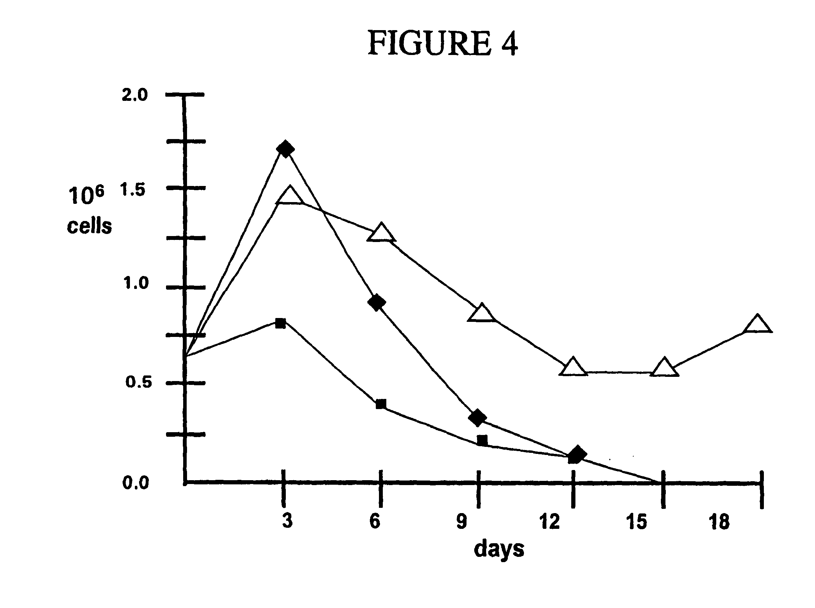

Figure 4 shows G418 resistance by infected monocytes. Freshly isolated adherent cells were treated with AAV/GM-CSF/Neo virus, exogenous GM-CSF, or untreated as indicated. After 48 hours the cells were selected with 300 µgs G418. Viable cells were then counted at the indicated times post-infection. The symbol ■ indicates G418 selected, mock infected cells. ◆ indicates G418 selected, exogenous GM-CSF treated cells. Δ indicates G418 selected, AAV/GM-CSF/Neo virus (1 ml) infected cells. Note that only the AAV dAAV/GM-CSF/Neo virus infected cells were resistant to G418.

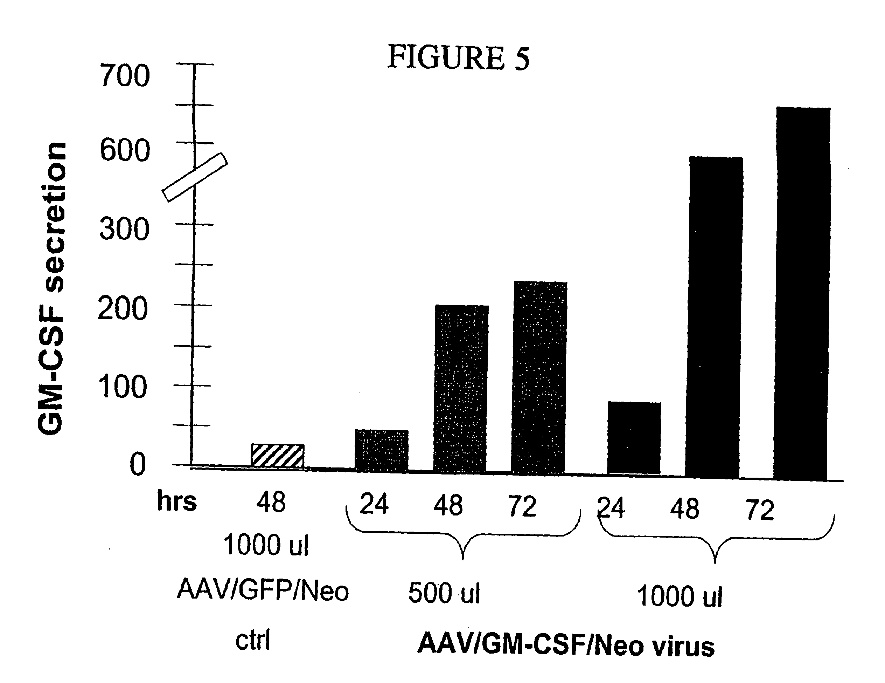

Figure 5 shows the secretion of GM-CSF by infected monocytes. Conditioned medium (100 µl from a total of 4ml) from the monocyte cell cultures was assayed by Sandwich enzyme immunoassay (EIA) for GM-CSF secretion for the presence of soluble GM-CSF protein as described below.

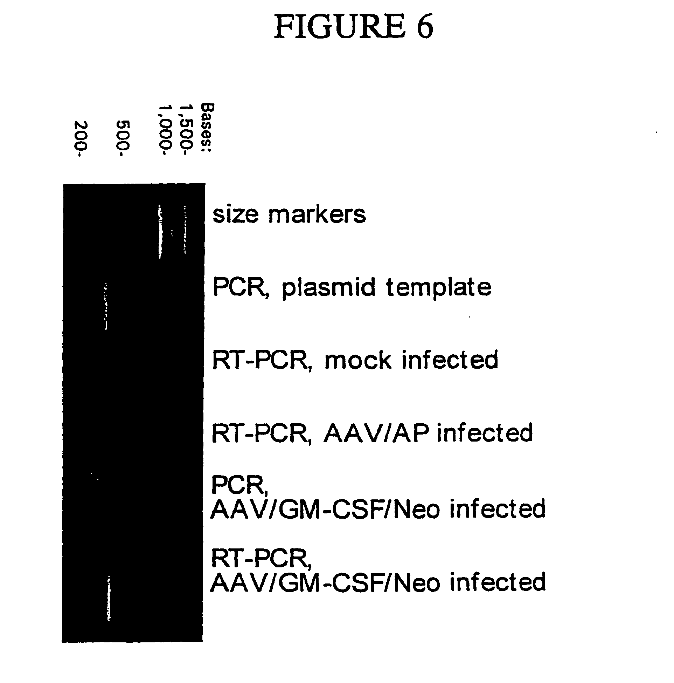

Figure 6 shows GM-CSF RNA expression in infected monocytes. Total RNA was isolated from three cell populations: mock infected, AAV-AP-infected, and AAV/GM-CSF/infected adherent monocytes at 48 hours post-infection. These samples were analyzed by RT-PCR and PCR, as indicated, for the presence of GM-CSF RNA as described below. The positive control was the PCR product resulting from using the AAV/GM-CSF/Neo vector plasmid as a template. Another control was PCR analysis of RNA from cells infected by AAV/GM-CSF/Neo virus.

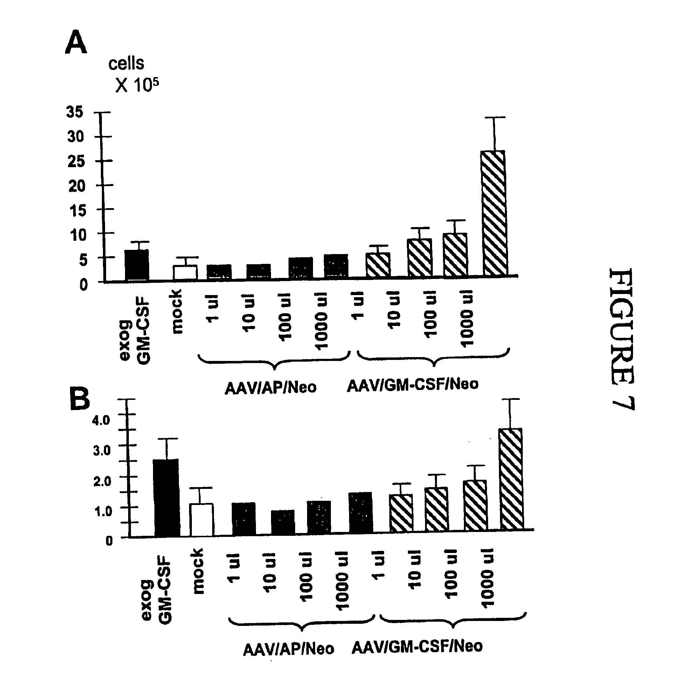

Figure 7 shows GM-CSF-induced monocyte proliferation by infected monocytes. Equivalent wells of freshly isolated adherent cells were treated as indicated, cultured for eight days, and then the cells counted. Figure 7A shows the total monocyte numbers generated by the indicated treatments. The experiment was done in quadruplicate, with mean and standard deviation given, with the exception of the AAV/AP/Neo infections which were done only once. Quantified cell numbers were: exogenous GM-CSF, 5.91 +/.67; mock. 3.42+/-.26; AAV/AP/Neo: 1 µl, 3.48; 10 µl, 0.3.74; 100 µl, 4.27; 1000 µl, 4.56; AAV/GM-CSF: 1 µl 4.98+/-.47; 10 µl, 5.75+/-.04; 100 µl, 7.07+/-.85_{:} 1000 µl, 25.31 +/-8.11. Figure 7B shows the number of cells of dendritic morphology (large cells with pseudopodia) generated by the indicated treatments. The experiment was done in quadruplicate, with mean and standard deviation given, with the exception of the AAV/AP/Neo infections which were done only once. Quantified cell numbers were: exogenous GM-CSF, 2.56+/-.73; mock, 0.95+/-.36; AAV/AP/Neo: 1 µl, 1.01; 10 µl, 0.82; 100 µl, 1.12; 1000 µl, 1.57; AAV/GM-CSF: 1 µl 1.083+/-.755; 10 µl, 1.377+/- .623; 100 µl, 1.67+/-.731; 1000 µl, 3.177+/-1.184

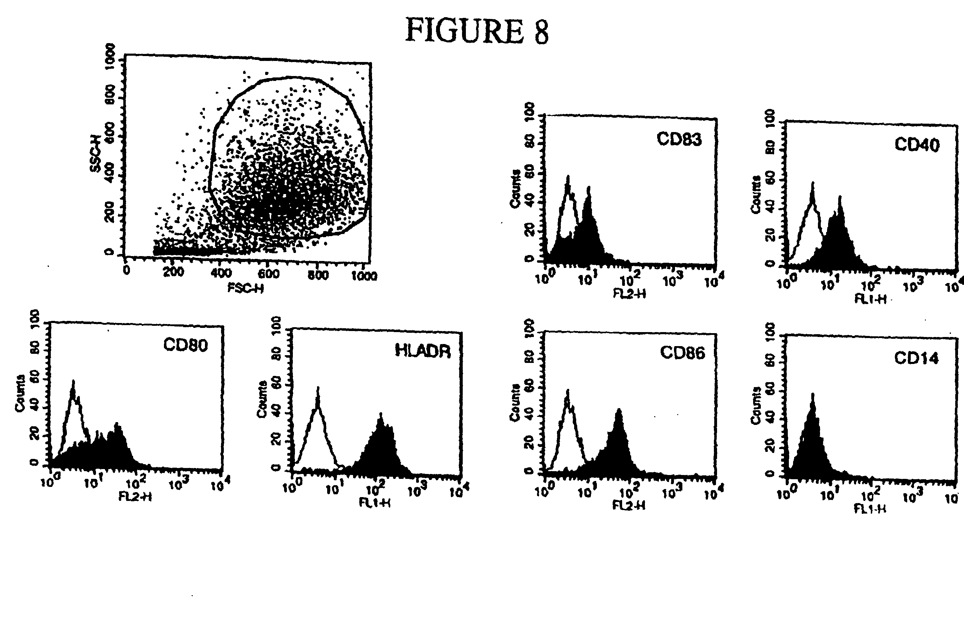

Figure 8 shows AAV/GM-CSF/Neo- infected monocytes differentiate into DCs, displaying multiple classical surface markers. Adherent monocytes were infected with with AAV/GM-CSF-Neo virus (1 ml) then, after 2 days, treated with IL-4 for four days. Finally the cells were treated with TNFα for two days. On day eight the cells were analyzed for the expression of HLA-DR (MHC class II)(97%), CD14 (0%), CD40 (74%), CD80 (64%), CD83 (37%), and CD86 (96%) by FACS analysis, as described below. Dendritic are DR+, CD40+, CD80+, CD83+, CD86+, and CD14-. Thus, the FACS profiles are consistent with the AAV/GM-CSF/Neo infected cells being mature DCs.
Figure 9 shows viral chromosomal integration in CD83 positive DCs. Cells treated as in Figure 6, and sorted for CD83 expression, were further analyzed for chromosomally integrated AAV/GM-CSF/Neo viral genomes. Traditionally generated DCs (exogenous IL-4 and GM-CSF addition) served as uninfected controls. Total cellular DNA (0.1 µgs) from the infected, CD83 + selected cells and uninfected cells served as template in PCR amplification assays using primers targeting the SV40 early promoter of the vector and the cellular repetitive Alu I element. The products were Southern blotted and probed with ³²P-Neo DNA. The positive control lane contained 100 ng of linearized AAV/GM-CSF plasmid (6.9 Kb). The negative control lane contained products from a PCR reaction with no template DNA.

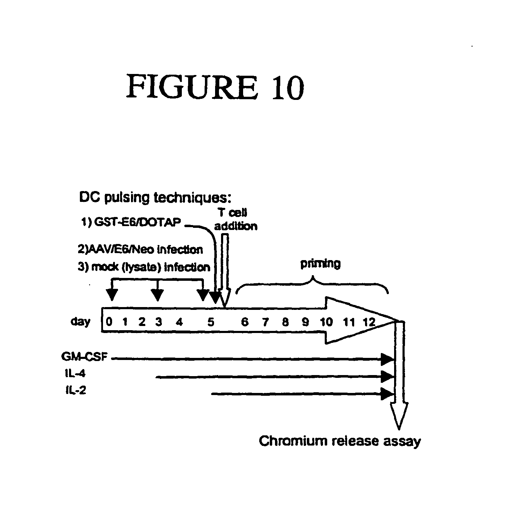

Figure 10 shows a diagram of the structure of the DC pulsing techniques used in the present invention.

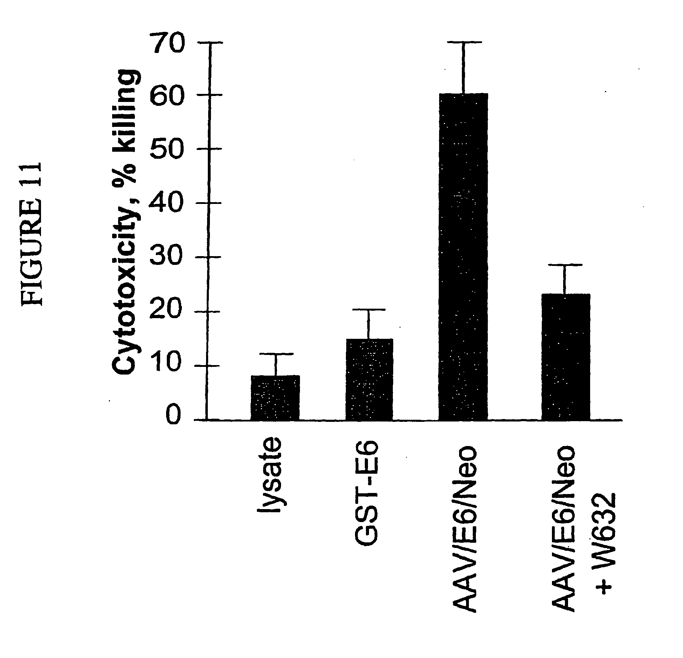

Figure 11 shows HPV-16 E6-specific CD8+ CTL response with DCs pulsed with the E6 gene in an AAV vector compared with pulsing DCs with bacterially produced E6 protein.

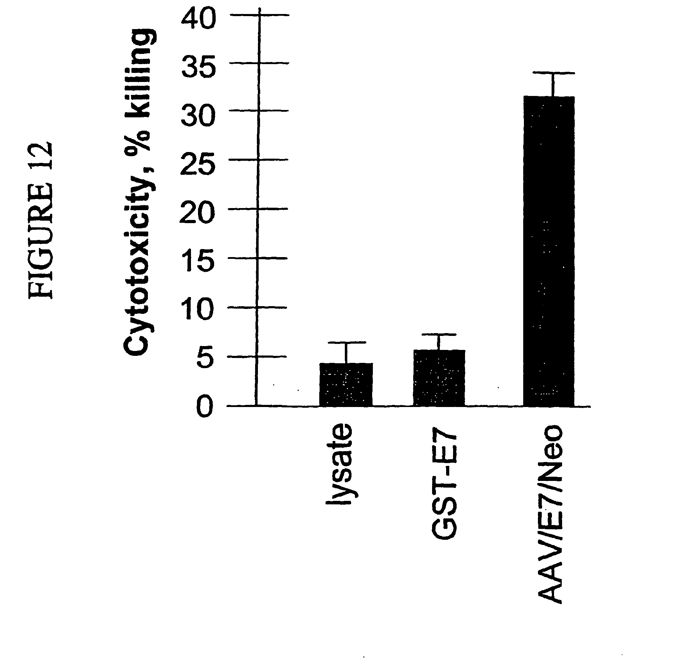

Figure 12 shows HPV-16 E7-specific CD8+ CTL response with DCs pulsed with the E7 gene in an AAV vector compared with pulsing DCs with bacterially produced GST-E7 that had been mixed with DOTAP.

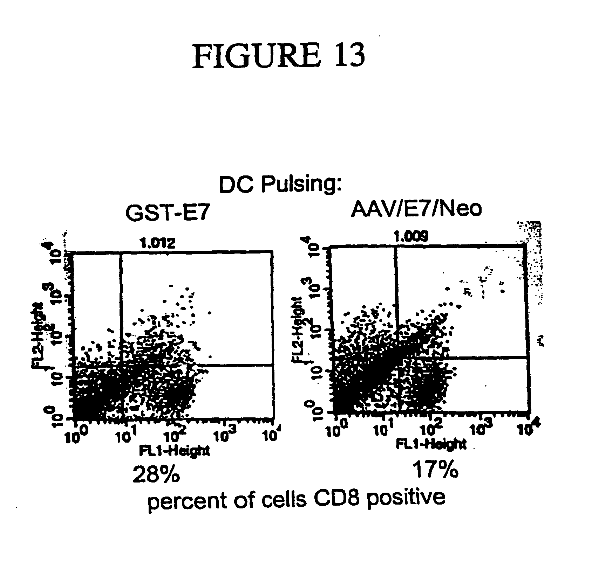

Figure 13 shows the FACS analysis of T cell populations resulting from different DC pulsing with DCs pulsed with the E7 gene in an AAV vector compared with pulsing DCs with bacterially produced GST-E7 that had been mixed with DOTAP.

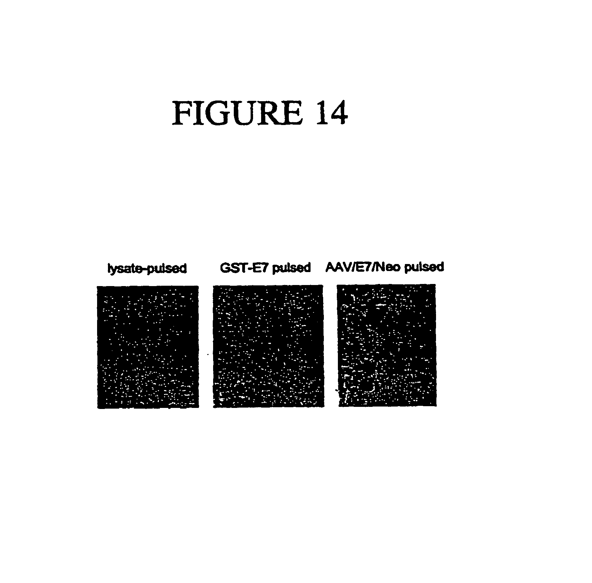

Figure 14 shows the early onset of DC-Tcell priming rosettes formed in AAV/E7/Neo pulsed DC-Tcell incubation compared to GST-E7 and lysate pulsing.

### DETAILED DESCRIPTION OF THE PREFERRED EMBODIMENT

The present invention provides. DCs that have been genetically altered by transduction with at least one AAV vector containing at least one heterologous gene. The term "transduction" refers to the process by which eukaryotic cells take up foreign DNA, and in some instances, integrate that foreign DNA into their chromosomes. The term "transfection" has a similar meaning to refer to the incorporation of foreign or heterologous DNA into eukaryotic cells. The success of this transduction indicates that ' AAV is an effective vector for manipulating DCs. There have been no reports of then successful transduction of DCs with AAV containing a heterologous gene where the AAV and heterologous gene are stably integrated into the cell's genome. The present invention is useful for generating DCs, particularly when the heterologous gene transduced into the DC, precursor cells via AAV transduction is a cytokine that promotes cell proliferation, e.g. GM-CSF. Because DCs are very efficient APCs, transducing them with heterologous genes provides a research tool for studying the function and the role these cells play in the immune response and a means for stimulating the immune response. The introduction and over-expression of immunologically relevant surface proteins provides the DCs with the capability to become an even better APC. For example, the over-expression of β2 microglobulin may result in improvements of MHC class I expression and improvements in dendritic CD8 cytotoxic T cell interaction and antigen presentation because of its role in regulating class,I levels (82-8,4). Additionally, the transduction and stable expression of the antigen gene within the DC itself improve DOC function. For example, when the heterologous gene is integrated in the DC and expresses one or more antigens and/or cytokines, such expression is a continuous source of the antigen or cytokine that is not lost or degraded when the DC is pulsed or incubated with these proteins. The heterologous gene also can be expressed, even if it is not integrated into the DC genome but exists in episomal form in the DC.

The present invention provides a method for preparing genetically altered DCs. wherein the method comprises: (a) obtaining DC precursors from a human blood sample; (b) preparing adherent dendritic cell precursors from said dendritic cell precursors and (c) transducing the DC precursors with at least one AAV vector comprising at least one heterologous gene to obtain genetically altered DCs expressing the heterolgous gene. The method of producing genetically altered DCs, more specifically comprises incubating the transduced precursors in medium with or without at least one cytokine that promotes cell proliferation of the transduced precursors. If there is no cytokine that promotes cell proliferation present in the medium, then at least one of the heterologous genes must encode at least one cytokine that promotes cell proliferation. In the presence of a cytokine that promotes cell proliferation, the DC precursors divide and proliferate during which time period, the AAV vector and its included heterologous gene(s) are preferably being integrated into the dendritic cell's genome. This cytokine can be any cytokine that promotes cell proliferation but GM-CSF is preferred. Then a second cytokine that promotes differentiation of the transduced dendritic cell precursors is added to the medium. Although any cytokine that promotes differentiation can be used. IL-4 and tumor necrosis factorα (TNFα) or a combination of the two is preferred, but IL-4 is most preferred. This cytokine that promotes cell differentiation is added sequentially after incubation with the cytokine that promotes cell proliferation, and not at the same time immediately after transduction as the first added cytokine that promotes cell proliferation. It has been shown that the longer the DC precursors are cultured in GM-CSF alone before adding IL-4, the less number of cells that differentiate into DCs.(64) The data show decreasing numbers of differentiated DCs after adding IL-4 at 2 days,than at 5 days and than at 9 days. Therefore, the optimum time period to add the IL-4 is at least at 2 days after transduction. However, culture conditions may vary, thus an adjustment of the timing to add IL-4 may vary from the 2 day optimum. The addition of IL-4 at least 2 days after transduction provides consistent results for converting maximum numbers of precursors to DCs. After the IL-4 is added the DC precursors, these precursors are preferably incubated for an additional 3 days to obtain DCs. These DCs can additionally be incubated for another 2 days with TNF-α, IL-1β, and PGE2a (prostaglandin E2a) to produce the final maturation of the DCs.

The AAV vector contains at least one heterologous gene that expresses at least one cytokine that promotes cell proliferation of the transduced dendritic cell precursors. Thus, the DC precursors transduced with a gene encoding at least one cytokine expresses a cytokine that is important to the cell for cell proliferation. The stable introduction of cytokine genes into the DCs offers an improvement over adding the cytokines exogenously and may benefit from the continuous production of cytokines from the cells. The medium for incubating the precursors after transduction can optionally contain a cytokine that promotes DC proliferation, such as, GM-CSF, to accelerate the cell proliferation stage of the incubation. The transduced cells alternatively are cultured in the absence of exogenous GM-CSF until the transduced cells produce their own GM-CSF, which is approximately 24 to 48 hours after transduction, and is in sufficient quantities to promote DC proliferation of the transduced precursors in the medium. Generally, it takes from 1 to 2 days for a newly integrated heterologous gene to be expressed in a mammalian cell. Then at least one cytokine that promotes differentiation of the DC precursors, such as IL-4, is added after transduction, using the time frame for addition discussed above, with addition of IL-4 preferably at approximately 2 days. Selecting the optimum time period for the addition of the cell differentiating cytokine is determined by the skilled artisan by trial and error experimentation. The minimum time for addition of the DC differentiation cytokine depends on a number of variables but can be affected by the addition of exogenous GM-CSF, in addition to the expression of heterologous GM-CSF from the transduced DC pecursors. The term " heterologous" is intended to mean that the source of the gene or protein is not from the specific cell that it has been introduced into.

The AAV transduced DC precursors are initially incubated in a cytokine to promote cell proliferation or in media without the cytokine to promote cell proliferation, in the case where the transduced DC precursors express their own cell proliferation cytokine or the DC precursors can produce their own cytokine and be incubated in medium containing the cytokine as well. This initial incubation that allows DC proliferation is followed by subsequent addition of at least one cytokine that promotes cell differentiation. Additionally other cytokines, such as TNF-α, IL- 1β and PGE2a can be added to obtain a fully mature DC beyond the incubation with GM-CSF and IL-4. With these steps, the following AAV vector constructs for precursor transduction are preferred but the invention is not intended to be limited to these constructs: AAV-E6, AAV-E7, AAV-L1, AAV-E6-E7, AAV-E6-E7-L1, AAV-BA46, AAV-Her-2, AAV-CD24, and AAV-any combination of any other disease specific, pathogen specific or other tumor specific antigen encoding genes, AAV-GM-CSF, AAV-IL-12, AAV-IL-4, AAV-GM-CSF-IL-12, AAV-GM-CSF-IL-4, AAV-GM-CSF-IL-12-IL-4 , AAV-any other cytokine or combination of cytokines and antigens, AAV-E6 or E7-GM-CSF, AAV-E7-IL-12, AAV-E6-IL-12, AAV-E6-E7-IL-12, AAV-GM-CSF-any other disease specific, pathogen specific or tumor specific antigen encoding genes, and AAV-any combination of antigens and cytokine genes not exceeding 5.5 kilobases in size. The goal is to obtain DC proliferation to a specified number and then add or turn on expression of the cytokine that promotes cell differentiation into a mature DC. The AAV vectors that contain IL-4 can be used but should be monitored for early differentiation of the DCs. Assuming that the integrated IL-4 gene will not be expressed for 1 to 2 days after transduction, the timing of the expression of IL-4 from the integrated gene(s) should allow earlier cell proliferation by the GM-CSF prior to the expression of IL-4. It also is possible to transduce the DC precursors with two separate AAV vectors, one containing the proliferation cytokine gene and the other one containing the differentiation cytokine gene. The formed vector transforms the DC precursors and then the latter vectors transforms the same cells after an appropriate period of time to allow sufficient proliferation of the cells prior to differentiation.

Alternatively, when the precursors are transduced with an AAV vector containing a cytokine that promotes cell proliferation, such as GM-CSF and a second cytokine that promotes cell proliferation, the following AAV constructs are useful: AAV-E7-GM-CSF, AAV-E6-GMCSF, AAV-L1-GM-CSF, AAV-GM-CSF, AAV-IL-12-GM-CSF, AAV-IL-4-GM-CSF, AAV-any other cytokine-GM-CSF, AAV-E7-E6-GM-CSF, AAV-E6-E7-L1-GM-CSF, AAV-E6-E7-IL-12-GM-CSF, AAV-any combination of antigen and cytokine genes not exceeding 5.5 kilobases in size.

Ideally, to prepare a DC that is useful to stimulate the immune response or study it, an AAV vector contains the AAV-GM-CSF- any pathogen specific, disease specific or tumor specific genes so that the DC precursors will proliferate with the expression of GM-CSF in the DC precursor and/or GM-CSF in the medium, and then at least one cytokine that promotes DC differentiation is added to the media. Also useful in the present invention is a DC that expresses a cytokine on a continuous basis after it has matured into a DC. To obtain such a cell, an AAV-GM-CSF vector transduces the DC precursors, which are then incubated with or without at least GM-CSF in the medium. At a predetermined time, a cytokine that promotes DC differentiation is added to the medium or an AAV-IL-4 gene containing vector transduces the DC precursors. To fully mature the DCs into more effective APCs, additional cytokines, such as TNF-α, IL-1β and PGE2a also are added to the media after the differentiation of the DC precursors by IL-4. Alternatively, an AAV vector containing at least one or preferably all of genes encoding TNF-α, IL-1β and PGE2a transducers the DCs to obtain fully mature DCs. The cytokines that are added to the medium to differentiate the DC precursors alternatively may be added by transducing the DC precursors with AAV vectors that encodes these DC differentiation cytokines so that these cytokines are expressed after the DC precursors have proliferated. The mature DCs expressing only cytokines, such as GM-CSF, IL-2 or IL-12, are useful as adjunct therapy for cancer treatment or for infectious disease treatment. These latter DCs would be useful to stimulate the basal level of tumor cell recognition.

The method for preparing genetically altered DCs comprises: (a) obtaining DC precursors from a human blood sample; (b) preparing adherent dendritic all precursors from said dendritic cell precursors (c) transducing the DC precursors with at least one AAV vector comprising at least one heterologous gene selected from the group consisting of a gene encoding a protein or antigen, a gene encoding a cytokine and combinations of one or more genes thereof; and (d) incubating the DC precursors in medium under conditions whereby the DC precursors differentiate into DCs that contain the heterologous gene stably integrated into the genome of the DC to obtain genetically altered DCs. As discussed above, the medium of incubation step (d) comprises at least one cytokine that promotes cell proliferation of said precursors, and wherein a second cytokine that promotes differentiation of the precursors is added to the medium subsequent to the addition of the first cytokine. The timing of addition of the cytokines and the specific cytokines are discussed above. As discussed above, the differentiation DCs also can be made available to the DC precursors by transduction with at least one AAV vector containing the genes encoding one or more of the DC differentiation cytokines.

The heterologous gene(s) contained in the AAV vector are operably linked to a promoter functional in the DCs. If more than one heterologous gene is present in the AAV vector, each heterologous gene is operably linked to a promoter functional in DCs or these genes can be connected as a polycistronic message under the control of a single promoter. Suitable promoters are known to persons skilled in the art. The heterologous gene or genes may be operably linked to the AAV p5 promoter but any promoter functional in the DCs are preferred. A promoter isolated from the DCs is also preferred as a useful promoter in the present invention and is expected to increase expression of the genes to which it is operably linked.

When the heterologous gene is an antigen, such as an antigen gene associated with a human disease, the DCs are useful to study the function and role of DCs in the immune system as well as useful for immunotherapy. The AAV vector transduction of antigens allows for better HLA (human leukocyte antigen) class I presentation of tumor and viral antigens when compared to exogenous protein introduction. Such class I presentation is critical for the induction of viral and tumor specific cytotoxic T cell response.(4)

The antigen gene is preferably a diseas specific antigen, a tumor specific antigen gene or a pathogen specific antigen gene. The preferred antigen is a human papilloma virus antigen, a hepatitis virus antigen, such a hepatitis B or C virus antigen, a HIV antigen and a breast cancer specific antigen. The more preferred antigens are HPV E6, HPV E7, HPV L1 capsid gene, and breast cancer specific antigens, BA46, Her-2/neu and CD24.

When the heterologous gene is a gene encoding a cytokine, the preferred cytokines are selected from the group consisting of a colony stimulating factor (CSF), interleukin (IL), such as IL-2, IL-4, IL-12, and IL-1,TNF,PG (prostatglandin) and combinations thereof. More preferably, the cytokines are GM-CSF, IL-4, IL-12, TNF α, IL-1β and PGE2a.

The preferred cytokine for generating DCs for studying and analysis are GM-CSF, IL-4, and TNFα, IL-1β and PGE2a. GM-CSF is added initially to promote cell proliferation and then IL-4, and if fully mature DCs are desired, then TNFα, IL-1β and PGE2 are added to obtain further cell maturation. When DCs are generated for use in stimulating the immune response of a subject, the GM-CSF, IL-2 and IL-12 are the preferred cytokines. The GM-CSF is preferably transduced into the cell precursors via AAV and the cell differentiation cytokines are added subsequent to the GM-CSF to obtain DCs for immunotherapy or priming the subject's T-cells.

The AAV vector used in the present invention is obtained by partial digestion of a wild type AAV genome with *Bsa*I to delete internal AAV sequences. More preferably the AAV vector contains a polylinker, and most preferably is d16-95/PL1. (139)

Genetically altered dendritic cell produced by any of the methods of the present invention are preferred cells but DCs encompassed by the present invention are any DCs comprising the AAV vector containing at least one heterologous gene, preferably stably integrated into the genome of DCs.

The present invention provides the use of genetically altered DCs, preferably the patient's DCs, comprising at least one AAV vector comprising at least one heterologous gene selected from the group consisting of a gene encoding an antigen associated with a disease afflicting the patient; a gene encoding a cytokine and combinations of one or more genes thereof in the preparation of a pharmaceutical composition for stimulating the immune system of a patient afflicted with a disease, whereby the heterologous gene is expressed in the DCs and the patient's immune system is stimulated by the stimulation of the patient's T cell response. As discussed above, genetically altered DCs are obtained by the previously described methods, and then are to be administered to the patient. The AAV vectors used to transduce the patient's DCs can contain one or more antigens associated with the patient's disease and/or one or more cytokine that promotes cell proliferation and cell differentiation. Additionally, more than one AAV vector containing the heterologous gene(s) can be used to co-transduce the DCs and the AAV vectors can be transduced at different times to allow the expression of different cytokines at different times in the development of the DCs. Several AAV vectors can be used to transduce the precursors as vehicles for bringing the desired heterologous genes into the cells. The antigen and/or cytokine genes become stably integrated into the genome of the DCs and are expressed in these cells but the AAV containing the genes may survive and be maintained in an episomal form. The dendritic cell precursors are obtained from the patient's blood by separating out the peripheral blood monocytes according to methods disclosed below and isolating adherent monocytes, also referred to a dendritic cell precursors.

It is believed that these genetically altered DCs provide for better HLA class I presentation of tumor or viral antigens, and thus better stimulate the patient's T-cell response. In regard to this immunotherapy, alternatively, the inclusion of a suicide gene encoding for example, herpes simplex type I thymidine kinase or a similarly functioning gene, would allow greater *in vivo* freedom for using DCs as they can be eliminated. Such techniques are known to persons skilled in the art. (85)

The use of the invention uses genetically altered DCs as described herein that contain the appropriate antigens for stimulating the patient's T-cell response, as well as cytokines necessary for the preparation of the DCs from their precursors. These genetically altered DCs are then to be administered to the patient to stimulate the patient's T cell response. Also described herein is the genetically altered DCs produced by the present method are incubated with the patient's CD8+ cytotoxic T cells prior to administering the genetically altered DCs to said patient.

Furthermore, the genetically altered DCs can be incubated with the patient's CD8+ cytotoxic T cells,and with or without the patient's CD4+ helper T cells to stimulate the T cells against the antigens that the genetically altered DCs are carrying. These stimulated T cells then are to be administered to the patient to stimulate the patient's T cell response.

The pharmaceutical composition in to be delivered to a mammal, e.g. a human subject or patient, by transducing dendritic cell precursors with an AAV vector containing at least one heterologous gene of the present invention and introducing the transduced cells into the mammal. The heterologous gene(s) are expressed in the genetically altered DCs, and in turn, stimulates the subject's T cell response. The genetically altered DCs can be incubated with the subject's CD8+ cytotoxic T cells, and optionally, to provide a stronger response, with the subject's CD4+ helper T cells. After this stimulation, the genetically altered DCs are separated from the T cells or the DCs and the stimulated T cells are to be administered to the subject in a pharmaceutical composition. The patient is parenterally administered a therapeutically effective amount of genetically altered DCs and/or CD8+ cytotoxic T cells and/or CD4+helper T cells in a pharmaceutically acceptable carrier pursuant to methods well known in the art, e.g. in a suspension of physiological PBS. The cells are to be administered intravenously, subcutaneously or directly into a tumor, however, the mode of administration can be any mode by which the cells are delivered to the patients body in an efficient manner to allow more efficient and effective delivery of the pharmaceutical composition. Dosages and frequency of administration varies with the age, weight and physical condition of the patient, accordingly to well-known protocols.

The following specific methods for carrying out the present invention exemplify the invention but are not meant to limit the scope of the invention to these specific examples:

### EXAMPLES

### MATERIALS AND METHODS

**Generation of Recombinant AAV Virus Stocks**.The wild type AAV genome pSM620 (54) was partially digested with *Bsa* I so as to delete the internal AAV sequences from map units 6 to 95 (nt 286-4460) and a specially designed polylinker was ligated in place, resulting in the AAV vector plasmid d16-95/PL1. (139) The AAV vector d16-95 is a BsaI-BsaI deletion vector with an intact p5 promoter. Into this polylinker the Neomycin resistance gene (Neo) and human granulocyte macrophage-colony stimulating factor (GM-CSF)(ATCC) gene were sequentially ligated. In the resulting plasmid, d16-95/GM-CSF^{p5}/Neo^{sv40} (hereafter referred to as AAV/GM-CSF/Neo), the GM-CSF gene was expressed from the AAV p5 promoter while Neo was expresses from the SV40 early promoter which was present on in the Neo fragment. The AAV/AP/Neo and AAV/GFP/Neo have been described before.(55,56) AAV vectors that utilize different promoters utilize the basic AAV vector d13-94, which has the p5 promoter deleted.

**Generation of high-titer rAAV virus stocks.** High titer virus stocks were generated in a two step process. To generate an initial virus stock 4 µgs of the AAV vector plasmid was lipofected with 0.5 µgs of the large wild type-plus AAV complementor plasmid ins96-0.8 (57) into adenovirus infected 293 cells (multiplicity of infection [MOI] of 5). After 24 hours the medium was removed and replaced with AIM-V medium (Gibco/BRL, Grand Island, NY). This virus stock was then used to generate high producer cell lines. To do this 293 cells were infected with the initial virus stock (0.5 ml) and then placed under G418 selection (600 µgs/ml). After 10 days resistant colonies were picked and expanded, with continuous G418 selection. Equivalent numbers of cells from each producer clone were compared for their ability to generate replicative AAV/GM-CSF/Neo DNA. The cells were infected with adenovirus (MOI 5) for two hours. Then, 0.5 µg of ins96-0.8 plasmid was DEAE-dextran transfected into the cells. After 30 hours DNA was isolated by the method of Hirt (58), and the levels of replicating vector DNA analyzed by agarose gel electrophoresis and Southern blotting (59) using ³²P-labeted Neo DNA as a probe.

To generate high titer AAV/GM-CSF/Neo virus stocks, the best producer cell line was infected with adenovirus at an MOI of 5 for two hours and then lipofected with 1 µg of ins96-0.8 plasmid. After 12 hours the medium was replaced with AIM-V medium without fetal bovine serum. The purpose of this step was to limit the amount of serum proteins in the virus stock, and to generate a virus stock which was compatible with the medium used to grow the DCs. After 48 hours the cells were frozen and thawed three times to lyse the cells, heated to 56°C to kill adenovirus helper, and filtered to remove cellular debris. The virus stocks were then frozen at -80°C.

To titer the virus stock 10 µls were first treated with 100 units of DNase I/ml for 1 hour (Sigma Corp. , cat. # D5025)(to destroy unencapsidated viral genomes). The Dnase I was then inactivated by heating to 65°C for 30 minutes. To isolate the virion DNA the virus stock was then digested with Proteinase K (0.5 mg/ml), phenol extracted, and ethanol precipitated. A series of known standardized amounts of AAV/GM-CSF/Neo plasmid, plus the DNA isolated from the virus stock, were then applied to a nylon membrane. The dot blotting was carried out as described previously.(60) Briefly, the DNA, in 5 µls of water, was first denatured by the addition of 10 µls of 0.4 N NaOH, incubated for 10 minutes, re-neutralized by the mixing of 200 µls of Tris/HCI, pH 7.0, then immediately adding the solution to a dot blot apparatus under suction. The blot was then probed with ³²P-labeled GM-CSF DNA. Comparing the signal strength of the unknown virus stock with the known standards determined the titer in encapsidated genomes/ml. Equivalent high titer virus stocks of AAV/AP/Neo and AAV/GFP/Neo were also generated in a similar manner as for AAV/GM-CSF/Neo.

**Separation and culture of monocytes and DCs from blood.** The peripheral blood mononuclear cells (PBMCs) were separated by routine Ficoll gradient method from fresh blood drawn from a healthy person. The PBMCs were inoculated into six-well culture plates and incubated with two milliliters of AIM-V medium for two hours at 37°C and 5 % CO₂. At that time non-adherent cells were removed by carefully washing the monolayer three times with phosphate buffered saline (PBS, pH 7.0). To induce differentiation of the adherent monocytes into DCs the cells were treated with human GM-CSF (LEUKINE^{®}, Immunex Corporation, 1.4x10⁶ units/250 µgs) at a final concentration of 800 IU/ml and human interleukin 4 (IL-4, R & D SYSTEMS company) at 1000 IU/ml for two-four days.(2.3) These control cells were then fed every two days by the addition of one ml of AIM-V medium with GM-CSF and IL-4 as indicated. A similar and alternative method of obtaining DCs from the blood is described in reference 139.

**Full Maturation of Dendritic Cell Cultures and Generation of HPV E7-specific T Cells via protein pulsing with HPV oncoproteins.** Heparinized peripheral blood (PBMC) was collected from two healthy adult donors under protocols approved by the Institutional review board. Adult healthy volunteers studied had no prior history of clinical or cytological HPV infection and a normal routine cervical smear (ie., donor 2) collected at the time of the study. The derivation of dendritic cells from the donor PBMC, and their subsequent use for generation of HPV E7-specific T cells, was carried out essentially as described. Briefly, between 40 to 50 ml of PBMC were placed into 6-well culture plates (Costar, Cambridge, MA) in AIM-V (Gibco-BRL) at 0.5-1 X 10⁷/3 ml per well. After 2h at 37°C, nonadherent cells were removed, and the adherent cells were cultured at 37°C in a humidified 5 % CO₂/95% air incubator, in medium supplemented with recombinant human GM-CSF [(800 U/ml), Immunex, Seattle, WA] and IL-4 [(1000 U/ml) Genzyme, Cambridge, MA]. Final maturation of monocyte-derived DC was induced by exposure during the last 48 hr. of culture (i.e. day 6 to day 8) to TNF-α (1000 U/ml) IL-1β (500 U/ml) (Research and Diagnostic, Minneapolis, MN) and PGE2a (0,5 M/M1) (Sigma, St. Louis, MO). After final maturation, the mature DCs were harvested for pulsing with E7 HPV 16 or 18 oncoproteins generated using previously characterized plasmids encoding glutathione S-transferase (GST) E7 fusion protein as described in reference 100. The cationic lipid DOTAP (Boehringer Mannheim. Indianapolis, IN) was used to deliver the HPV 16 or 18 E7 proteins into cells as described. Generation of HPV-E7-specific CTLs was obtained culturing responder PBMC (10-20x10° cells/well in 6-well culture plates) (Costar) in AIM-V with E7 pulsed autologous DC (ratios from 20 : 1 to 30 : 1 responder PBMC:DC). The cultures were supplemented with recombinant human IL-2 (10 U/ml-Aldesleukin. Chiron Therapeutics, Emeryville, CA) and restimulated once with E7-pulsed DC after 10-14 days. At day 21-28, DC8+T cells were separated from the bulk cultures by positive selection with CD8-Dynabeads (Dynal Inc., Lake Success, NY) and further expanded in number for 5-7 days using autologous or allogeneic irradiated PBL (5000 cGy) (1x10⁶ cells/well) and anti-CD3 monoclonal antibody (Ortho Pharmaceutical Corp, Raritan, NJ) (0.2 µg/ml) plus 5% autologous plasma and 100 U/ml of IL-2 in 24-well plates (Costar), before being assayed for phenotype characteristic and CTL activity. In some experiments, to directly assay and differenziate the cytolitic capacity as well as the stability of the phenotype characteristic of the two populations of CD8+ effector T cells obtained (i.e., CD8+/CD56+ and CD8+/CD56-), sorted fractions (see below) were cultured in RPMI 1640 plus 5% autologous plasma and 100 U/ml of IL-2 in 24-well plates for different time, before being assayed for phenotype by flow cytometry or for CTL activity.

**Infection of monocytes with AAV virus.** Immediately after the removal of the non-adherent cells, the adherent monocytes were infected with the indicated amount of virus stock. After two hours incubation the medium/virus solution was removed, the cells were washed with AIM-V, and finally fed with AIM-V medium.

**Sandwich enzyme immunoassay (EIA) for GM-CSF secretion.** Medium (100 µl from a total of 4ml) from the monocyte /dendritic cell cultures was assayed for the presence of soluble GM-CSF protein by using the *ChemKine* Human Granulocyte Macrophage-Colony Stimulating Factor kit (Chemican International, Inc.) as directed by kit instructions. The results were then mathematically extrapolated to give total GM-CSF secretion (x40).

**Monocyte proliferation analysis.** For the monocyte proliferation assay no exogenous GM-CSF or IL-4 were added to the experimental AAV/GM-CSF/Neo or AAV/AP/Neo virus infected cells. In other wells, when indicated, control cells were treated with human GM-CSF at a final concentration of 800 IU/ml and/or IL-4 at 1000 IU/ml. The growth of the control and infected cells was measured for 18 days by removal of 500 µls of medium after the resuspension of the cells, and counting cells with a hemocytometer in conjunction with trypan blue exclusion.

**Determination of G418 resistance.** When G418 resistance was measured 300 µgs of G418 per ml was added at 2 days post-infection and for the remainder of the experiment. The survival and growth of the cells resistant to G418 was measured by counting cell numbers with a hemocytometer in conjunction with trypan blue exclusion.

**Reverse transcription-polymerase chain reaction (RT-PCR) analysis of RNA Expression.** Monocytes were first infected with AAV/GM-CSF/Neo virus (1ml). Control cell populations included mock infected cells, and cells infected with AAV/AP/Neo virus. At 48 hours post-infection the cells were harvested and treated with TRIzol reagent (GIBCO BRL company) according to the protocol. Isolated total RNA was treated with RNase-free DNase I (Promega company) 3 units/ml for 2 hours at 37°C, and then redissolved in DEPC-treated water. Total RNA was then denatured for 10 minute at 70°C and then chilled on ice. First-strand cDNA synthesis was performed from 1 µg of the total RNA at 42°C for 1 hour in a final volume of 25 µls (10 µl of denatured RNA, 5 µl of 5X reaction buffer 250 mM Tris-HCI (pH 8.3), 375 mM MgCl₂, 15 mM KCI, 50 mM DTT), 300 units of Moloney murine leukemia virus (M-MLV) reverse transcriptase (Promega), 5 µl each of the four dNTPs. (10 mM each), 0.5 µg of oligo (dT)₁₅ (Promega), and 30 units of RNasin (Promega). PCR amplification of the cDNA was performed in a 100 µl reaction volume, which contained 10 µl of 10X PCR buffer (100mM Tris-HCI, pH 8.3; 500 mM KCI; 15 mM MgCl₂; and 0.01 % gelatin), 80 µM of each dNTP (Promega), 1 µM of each primer, and 2.5 units of *Taq* DNA polymerase. Both PCR primers have been described and are designed to amplify a 381 base segment of the GM-CSF sequence (up-stream primer 5'TGCAGAGCCTGCTGCTCTTG, and down-stream primer 5'CAAGCAGAAAGTC CTTCAGG)(60). After 5 minutes at 94°C, each sample was subjected to following amplification cycle: 50 second at 94°C, 55 second at 58°C, and 1 minutes at 72°C, for 32 cycles, and then 10 minutes at 72°C. The positive control was AAV/GM-CSF/Neo vector. The second control was the total RNA treated with DNase I from the cells infected by AAV/GM-CSF/Neo virus. The others were the cDNA from the total RNA of mock-infected cells and the cells infected by AAV/AP/Neo virus.

**Cell surface marker analysis by flourescent antibody cell sorting (FACS).** Mature DCs were produced using AAV/GM-CSF/Neo virus in place of exogenous GM-CSF. To do this freshly drawn adherent monocytes were immediately infected with 1ml of AAV/GM-CSF/Neo virus. At days 2 and 4 IL-4 was added as above. At day 6, TNFα, at 1000 IU/ml, was added. The cells were then harvested at day 8 for analysis. A panel of mABs recognizing the following antigens were used: anti-DR, anti-CD14, anti-CD80 (Pharmingen, San Diego, CA), antiCD40 (Immunotech, Marseille, France), and anti-CD83 (Coulter, Miami, FL). Control irrelevant isotype-matched FITC- or PE- conjugated mAb were obtained from Becton-Dickinson. Briefly, cells infected with AAV/GM-CSF/Neo virus and uninfected controls, were harvested at day 8 of culture by washing the plates with PBS (pH7.2, Gibco/BRL). Adherent cells were recovered by incubating the plates at room temperature for 30 minutes in the presence of Ca⁺² and Mg⁺² free PBS containing EDTA (2 mM), followed by gentle scrapping. These cells were greater than 95% viable as assessed by trypan blue exclusion. Cell suspensions were counted and distributed into 12X75 mm tubes. Mouse monoclonal antibodies were diluted in cold assay buffer and the final pellet was resuspended in 500 µl volume. Tubes were incubated for 30 minutes followed by two washes with assay buffer and the final cell pellet was resuspended in 500 µls of assay buffer for subsequent analysis. Cells were analyzed with a fluorescence activated cell sorter (FACS; Becton-Dickinson) with a 15 mW argon laser with an excitation of 488 nm. Fluorescent signals were gated on the basis of cell dimension (i.e. forward and right angle light scattering typical of DCs. Gated signals (5,000-10,000) were detected at 585 BP filter and analyzed using Cell Quest software (Becton-Dickinson).

**Purification of CD83 positive and negative DCs by cell sorting.** To isolate specific CD83 positive DCs, equivalent cells as above (AAV/GM-CSF/Neo virus, IL-4 and TNFα treated) were harvested with rapid pipetting and sorted by FACSStarPlus (Becton Dickinson) using differences in dimension (high forward and side light scatter) as well as expression of the CD83 molecule. All the procedures of staining and sorting were performed in the presence of 5nM EDTA in order to avoid cell aggregation. Briefly, DCs were separated from contaminating lymphocytes, by incubating them in PE-conjugated CD83 on ice for 30 minutes and washed before cell sorting. The Gate was set for cells of the dimension of DCs (i.e. characteristic high forward and side light scatter), and two different populations were separated according to CD83 expression in CD83+ and CD83-. DCs positive and negative for the CD83 lineage specific marker were evaluated for integrated AAV/GM-CSF/NEO provirus.

**Detection of viral integration by PCR/Southern blot analysis.** To analyze for AAV vector chromosomal integration. DNA was isolated from the above CD83 + sorted/AAV/GM-CSF/Neo infected DCs and mock infected cells. Approximately 100,000 CD83+ cells used in this procedure. Total DNA was isolated from the cells by pelleting the cells and resuspending them in lysis buffer (5 mM Tris/HCL (pH 7.4), 5 mM EDTA, 0.5 mg proteinase K/ml). After incubation for at least four hours the total cellular DNA was phenol extracted twice and ethanol precipitated twice. Amplification of DNA samples was carried out in 100 µl reactions using approximately 0.1 µgs of total cellular DNA, 2 mM of each dNTP; 1 µM of each primer and 2.5 U of *Taq* DNA polymerase according to the supplier's (Fisher Scientific Company) instruction, After 5 minutes at 94°C, each sample was subjected to following amplification cycle: 50 second at 94 °C, 1 minute at 60 °C and 2 minutes at 72 °C, for 30 cycles, and then 10 minutes at 72°C. Primer 1 was complementary to the SV40 early promoter ( 5'-GCAGGCAGAAGT ATGCAAAG-3'). Primer 2 (5'-GAATTCAGCACAAATTGTAG), previously characterized by Batzer *et al* (1991)(61), was complementary to a common human repetitive element, *Alu* I. After electrophoresis, the reaction products, along with sizing marker DNAs, were directly visualized by ethidium bromide staining and UV fluorescence. The gel was then Southern blotted and probed with P³²-Neo fragment DNA from pBR-Neo.(63)

**Generation of CD4+ and CD8+ T cell population:** CD8+ cells are separated from the bulk cultures by positive selection with CD8-Dynabeads (Dynal Inc., Lake Success, NY) and further expanded in numbers for 5-7 days using autologous or allogeneic irradiated PBL (5000cGy, 1 x 10⁶ cells/well) and anti-CD3 monoclonal antibody (Ortho Pharmaceutical Corp., Raritan, NJ) (0.2 ug/ml) plus 5% autologous plasma in 24-well plates (Costar, Cambridge, MA), before being assayed for CTL activity. CD4+ T cells will be derived from day 21 CD8 + depleted T cell populations.

**Generation of autologous target cells:** Either cultured primary autologous tumor cells (from patients with cervical cancer) are prepared or target "HPV-infected" cells are generated by first immortalizing autologous B cells with Epstein Barr Virus and then lipofecting (DOTAP) or transducing (AAV) the antigen protein into these EBV transformed B cells. Patients who have undergone tissue removal provide tumor, tissue and peripheral blood lymphocytes. Specimens are obtained at the time of surgery or biopsy. In these examples, the antigenic protein may be proteins, such as HPV antigens (E6 or E7), specific breast cancer antigens (BA46, Her-2, CD24 or others), hepatitis virus antigens (hepatitis B or C virus antigens), HIV antigens or any other antigen that is linked to a cancer or infectious disease.

Fresh autologous tumor cells are maintained in serum-free keratinocyte medium (KSFM-Gibco/BRL), supplemented with 5 ng/ml epidermal growth factor and 35-50 ug/ml bovine pituitary growth extract (Gibco Life Technologies) at 37°C, 5% CO₂. Briefly, single-cell suspensions are obtained by processing solid tumor samples under sterile conditions at room temperature. Viable tissue is mechanically minced in RPMI 1640 to portions no larger than 1-33 mm³ and washed twice with RPMI 1640. The portions of minced tumor are placed into 250 ml trypsinizing flasks containing 30 ml of enzyme solution (0.14 % Collagenase Type I [Sigma], and 0.01 % DNAse [Sigma, 2000 KU/mg]) in RPMI 1640, and incubated on a magnetic platform overnight at 4°C. Enzymatically dissociated tumor is filtered through 150-um nylon mesh to generate a single cell suspension. The resultant cell suspension is then be washed twice in RPMI 1640 plus 10% autologous plasma. All experiments are performed with fresh or cryopreserved tumor cultures, which have at least 90% viability. The natural killer (NK) sensitive target K562 (a human erythroleukemia cell line), obtained from the American Type Culture Collection (ATCC), also is used.

**Generation of purified protein for DC pulsing:** Large amounts of purified proteins are produced using previously characterized plasmids encoding glutathione-S-transferase (GST) antigen protein fusion proteins (100). Briefly, GST and derivative fusion proteins are maintained in *E*. *coli* BL21, and protein expression are induced in cultures at an optical density at 550 nm of 0.6 by addition of isopropyl-β-D-thiogalactoside (IPTG); final concentration, 0.1 mM. Cultures are grown for 6 hours for large scale preparations (2000-4000 ml). Cells are pelleted and washed once in ice-cold 1x PBS, and then resuspended for disruption using sonication on ice in short bursts. 20% Triton X-100 are then be added to a final concentration of 1 % and mixed gently for 30 minutes to aid solubilization of the fusion proteins. Bacterial lysates are centrifuged at 10,000 rpm for 10 minutes at 4°C and supernatant purified using a Glutathione Sepharose 4B RediPack column in accordance with procedures suggested by the manufacturer (Pharmacia Biotech, Inc., Piscataway, NJ). Cleavage of protein from GST are achieved using a site-specific protease (human thrombin, Sigma). The antigen protein is eluted from the column by centrifugation (3000 rpm x 5 minutes), and collected material is sterilize-filtered. Purity of the protein is analyzed by SDS-PAGE and Coomassie staining while quantification is obtained spectrophotometrically using the BIO-RAD protein assay (Bio-Rad Laboratories, Hercules, CA). Preparations were typically > 95 % pure protein is generated from bacterially produced GST-fusions (100).

**AAV virus infection and antigen protein and peptide pulsing of DCs:** rAAV infection usually takes place at the initial step of isolating adherent monocytes. This is because, in preliminary studies, cells still have some proliferative capacity which favors AAV integration/transduction (64). As described above, freshly adherent monocytes are infected with the indicated amount of rAAV. After 36 hours, the cells are treated with IL-4 and GM-CSF to generate standard DC cultures. For protein pulsing, following standard culture (as described above), DCs are washed twice in AIM-V and added to 50-ml polypropylene tubes (Falcon, Oxnard, CA). Proteins, such as full-length E6 and E7 proteins are used for pulsing. The cationic lipid DOTAP (Boehringer Mannheim, Indianapolis, IN) is used to deliver the antigenic proteins and peptides into cells. Antigenic protein (100 ug/ml) and DOTAP (125 ug in 500 ul of AIM-V) are mixed in 12 X 75 mm polystyrene tubes at room temperature for 20 minutes. The complex is added to the DC culture in a total volume of 2-5 ml of AIM-V and incubated at 37°C in an incubator with occasional agitation for 3 hours. The cells are washed twice with PBS and resuspended in AIM-V, as described below.

**Testing rAAV and protein pulsed DCs for stimulating CTL activity:** Fresh or cryopreserved responder PBMCs are washed and resuspended in AIM-V at 10-20 x 10⁶ cells/well in 6-well culture plates (Costar, Cambridge, MA) with rAAV or protein pulsed autologous DCs (ratios from 20-30:1, responders:dendritic). The cultures are supplemented with recombinant human GM-CSF (500 U/ml) and recombinant human IL-2 (10 U/ml). At day 14-21, CD8+ cells are separated from the bulk cultures by positive selection with CD8-Dynabeads (Dynal Inc., Lake Success, NY) and further expanded in number for 5-7 days using autologous or allogeneic irradiated PBL (5000cGy, 1 X 10⁶ cells/well) and 0.2 ug/ml anti-CD3 monoclonal antibody (Ortho Pharmaceutical Corp., Raritan, NJ) plus 5% autologous plasma in 24-well plates (Costar), before being assayed for CTL activity. As negative control targets, autologous lymphoblasts are prepared by 3-day stimulation with 1 ug/ml Con-A (Gibco/BRL) in RPMI 1640 plus IL-2 (25 U/ml). EBV-transformed autologous lymphoblastoid B-cell lines (LCL) are established by coculture of PBMCs with EBV-containing supernatant from the B95. 8 cell line in the presence of 0.5% ug/ml cyclosporin A (Sandoz, Camberley, UK). These are maintained in AIM-V supplemented with 10% human AB serum (Gemini Bioproducts, Calabasas, CA).

A 6-hour chromium-51 release assay are performed as previously described (Figure1, in reference 100), to measure the cytotoxic reactivity of DCs-antigen specific-stimulated T lymphocytes. Autologous cervical tumor cells, allogeneic partially HLA- and HPV-matched cell lines, and K562 tumor cells are used as targets. Autologous Con-A-activated peripheral blood lymphocytes and/or EBV-transformed autologous LCL also are used as autologous control targets. To determine the structures on the effector and target cells involved in lysis. monoclonal antibodies (Mabs) are used to block cytotoxicity. Effector cells are preincubated for 30 minutes at 4°C with Mabs, which recognize human CD3 (10 ug/ml) (Ortho Pharmaceutical Corp.) or anti-CD11a/LFA-1 (10 ug/ml) (Pharmingen, San Diego, CA) and its isotype control (IgG1K mAB isotype standard anti-TNP [10ug/ml] [Pharmingen]). The ⁵¹Cr-labeled tumor targets are preincubated with Mabs specific for monomorphic HLA class I W6/32 (50ug/ml) (hybridoma obtained from the ATCC, Rockville, MD). The effector cells and ⁵¹Cr-labeled targets are then incubated in a final volume of 200 ul for 6 hours at 37°C with 5% CO₂.

**T cell proliferation assay:** CD4+ T cells, derived from day 14-21 CD8+-depleted T cell populations, are re-stimulated once with rAAV or antigen protein-pulsed DCs, and 2 weeks later further purified by positive selection with CD4-Dynabeads (Dynal, Inc.) to obtain a >99 % pure population. Specific lymphoproliferative responses against E6 and E7 are tested using autologous LCL pulsed with full-length antigenic proteins, such as, BA46, Her-2, or CD24, in the presence of DOTAP, as described above for DC pulsing. For example, irradiated BA46-pulsed or unpulsed autologous LCL are seeded in a 96-well plate (2 x 10⁴ cells/well). CD4+ T cells (2 x 10⁴ cells/well) are tested for specific proliferation after 72 hours. Cultures are pulsed with 1 uCi/well of [³H]thymidine for the next 16 hours, and incorporated radioactivity measured as described (138).

**Cytokine release assay:** Autologous LCL pulsed with antigenic, or unpulsed (control), are cultured overnight with CD4+ T cell populations obtained after the different primings (AAV or DOTAP), after which supernatants are harvested by centrifugation and stored at -80°C until assayed for the presence of cytokines. Secreted cytokines are measured with commercially available ELISA kits for GM-CSF and INFγ (Research and Diagnostic Systems, Minneapolis. MN), with a detectable range of 8-500 pg/ml. Cytokine concentration are expressed as pg/ml/24 hrs. Secretion by CD4+ T cells in the presence of protein-pulsed LCL are considered significant if it exceeds secretion in response to control LCL by at least 2-fold.

Controls: Unpulsed, mock infected DCs serve as a negative control. The full-length antigenic protein-pulsed DCs serve as the positive and comparative control. Any of the experimental rAAV/antigen vectors that show stimulation of significant proliferation or cytokine secretion, particularly if better than antiagenic protein-pulsed DCs, are tested multiple times for reliability and to give quantitative results with standard deviation.

### RESULTS

**Monocytes infected with AAV/GM-CSF/Neo virus are G418 resistant.** Figure 1 shows the structure of the AAV/GM-CSF/Neo vector which was constructed for this study. To generate high titered AAV/GM-CSF/Neo virus stock, producer cell lines were generated as described in the Material and Methods section, and a Southern blot comparison of these cell lines is shown in Figure 2A. Clone 5 (293/AGN-5), which generated the highest level of replicating AAV/GM-CSF/Neo vector, was picked as the primary producer cell line. However, note that that there was a wide variety of rescue levels from these clones. The majority of clones rescued only low levels of the vector, and some did not rescue at even low levels. It is the inventors' hypothesis that these different rescue levels reflect different provirus stuctures, suggesting that a variety of proviral structures are possible for AAV. Infected 293/AGN-5 cells were then DEAE-dextran transfected with ins96-0.8 complementor plasmid, as described in detail in the Materials and Methods section, to generate stocks of AAV/GM-CSF/Neo virus for experimental use. The titering of the stock, by comparative dot blot hybridization, is shown in Figure 2B, and demonstrates that this stock was approximately 10¹¹encapsidated genomes per ml. Infectious units/ml were likely to be 0.1-1% of this (10⁸-10⁹ IU/ml). Virus stocks of AAV/GFP/Neo and AAV/AP/Neo were also generated in a similar manner.

To isolate a population of monocytes/dendritic cell precursors from blood the technique previously developed were used (2,3) (Figure 3, Method A). Briefly, mononuclear cells from fresh peripheral blood were separated from other cells by a Ficoll gradient and then plated into tissue culture plates for 2 hours. After the incubation period the non-adherent cells were gently washed away. Under these conditions the adherent cells have been found to be 60-90% of the dendritic cell type after the induction of differentiation by exogenous GM-CSF and IL-4.(64) The purity of the resulting dendritic cell population appears dependent upon the particular donor. DCs have little or no proliferation capabilities. However, the sequential addition of first GM-CSF for two days, followed by the addition of IL 4, as shown in Figure 3, Method B, does allow for a period of cell proliferation and still results in high levels of DCs.(64) It has been noticed in other cell types that AAV transduction is significantly higher in target cells which are actively dividing. (65-67) Therefore, the precursor cell type was infected and transduced before IL-4 exposure (Figure 3, Method C).

To demonstrate that the AAV transduction of the adherent monocytes was taking place, the G418 resistance assay was utilized as the vector containing the Neo gene which confers resistance to this reagent. Three experimental populations were included in this experiment: mock infected cells, mock-infected cells treated with exogenous GM-CSF, and cells infected with 1000 µls of AAV/GM-CSF/Neo virus. At two days post-infection the three populations were put under G418 selection at 300 µgs of G418/ml. At time 0, and at 2 day intervals thereafter, viable cell counts were taken. The results of one of three such experiments, all of which demonstrated AAV-mediated transduction of G418 resistance, is shown in Figure 4. Note that, as expected, significant initial cell proliferation took place in the exogenous GM-CSF and virally infected groups. G418 takes several days to begin killing cells. In all three groups total cell populations dropped. However, while the untreated and GM-CSF treated groups were largely killed by day 12, the AAV/GM-CSF/Neo virus infected group contained significant levels of resistant cells out to day 18. Thus, these data are fully consistent with the transduction of the Neo gene by the recombinant AAV vector.

**Monocytes infected with AAV/GM-CSF/Neo virus secrete GM-CSF.** The results showing G418 resistance were promising, thus we next observed if similarly treated monocytes also secreted GM-CSF. Monocytes were infected with either AAV/GM-CSF/Neo virus or AAV/GFP/Neo (control). The results (Figure 5) show that conditioned medium from control cells had only low levels of GM-CSF (28 pg total at 48 hours). Monocytes and DCs are known to express GM-CSF at low levels. In sharp contrast, monocytes infected with two different doses (500 and 1000 µls) of AAV/GM-CSF/Neo virus secreted significantly higher levels of GM-CSF (210 and 622 pg, respectively) at 48 hours. Thus, the AAV/GM-CSF/Neo virus vector appeared active for both the Neo and the GM-CSF genes and the AAV/GM-CSF/Neo infected cells secred much more GM-CSF than control cells.

**Monocytes infected with AAV/GM-CSF/Neo virus expressed GM-CSF RNA**. To further characterize the transduced GM-CSF gene activity the transcriptional activity of the GM-CSF gene(s) was analyzed by RT-PCR. Three cell populations, mock infected, AAV/AP/Neo infected, and AAV/GM-CSF/Neo infected, adherent monocytes populations were generated and total RNA was isolated at 48 hours post-infection. These RNAs were then analyzed by RT-PCR or PCR as described in the Materials and Methods section. The data, shown in Figure 6, demonstrates that GM-CSF RNA is being expressed, as indicated by an appropriate RT-PCR sized product; i.e.. a 381 bp fragment, only in those cells infected with AAV/GM-CSF/Neo virus. Analysis of RNA from cells mock infected or AAV/AP/Neo infected did not give this result, nor did simple PCR analysis of the RNA the the AAV/GM-CSF/Neo infected cells, indicating lack of contaminating DNA. Thus, these data further support the interpretation that AAV/GM-CSF/Neo virus is transducing the monocytes.

**Monocytes proliferate upon AAV/GM-CSF/Neo virus infection.** IL-4 contributes to monocyte differentiation and arrests cell proliferation. (2, 3, 64) Without IL-4, GM-CSF is monitored by functional activity shown by cell proliferation. (64) Monocytes were infected with increasing amounts of AAV/AP/Neo (control) or AAV/GM-CSF/Neo virus. These populations were then compared to one in which exogenous GM-CSF (800 µgs/ml) was added every other day. At time 0, and at 2 day intervals, viable cell counts were taken of all cells and cells of dendritic morphology (large cells with pseudopodia). As shown in Figure 7A for day 8, infection with AAV/AP/Neo did not result in significant increases in monocyte numbers, for either total cells or those of dendritic morphology. (64) In contrast, AAV/GM-CSF/Neo infected cells showed higher proliferation, and in a dosage dependent manner. Note that there are many more cells present in the exogenous GM-CSF treated culture compared to untreated and AAV/AP/Neo treated cells. Also note that the number of cells in the AAV/GM-CSF/Neo treated wells was directly dependent upon the amount of virus used, and that the cell numbers stimulated were higher those stimulated by exogenous GM-CSF addition and infection by AAV/AP/Neo. Further note that both 100 and 1000 µls of AAV/GM-CSF/Neo virus stimulated more monocyte proliferation than the exogenous GM-CSF control. Other time points gave similar results and counts of total cells also showed similar trends (data not shown). Thus, these data suggest that the transduction of the GM-CSF gene can fully replace the need for the addition of exogenous GM-CSF.

In the traditional protocols about 3,200 units, or 570 ng of exogenous GM-CSF are added to each of the cultures to stimulate differentiation or proliferation. In this experiment, 1 ml of virus, resulting in about 600 pg of GM-CSF secretion, gives an equal or superior effect. Thus, it is noteworthy that the activity of the recombinant GM-CSF appears to be one thousandth that of freshly secreted GM-CSF compared on a per weight basis.

Figure 7B shows the number of cells of dendritic morphology; i.e., large cells with pseudopodia, generated by the indicated treatments. Note that the number of cells in the AAV/GM-CSF/Neo treated wells was directly dependent upon the amount of virus used, and that the cell numbers stimulated were higher those stimulated by exogenous GM-CSF addition and infection by AAV/AP/Neo.

**AAV/GM-CSF/Neo virus infected monocytes give rise cells with classical dendritic surface markers.** The monocytes were analyzed to determine whether the introduction and stable expression of the GM-CSF gene causes alterations in DC phenotype, as when compared to the traditional technique adding exogenous GM-CSF to the culture. Thus the putative dendritic cell populations resulting from AAV/GM-CSF/Neo transduction were analyzed for a number of classical dendritic cell markers.(68) To generate these cells freshly adherent monocytes were infected with AAV/GM-CSF/Neo virus. At two days post-infection these cells were treated with IL-4 for 4 days. Finally, to induce the fully mature dendritic cell phenotype, the cells were treated with TNFα for 2 days.(69-73) On day eight the resulting cells were then analyzed by FACS analysis for HLA-DR (97%), CD14 (0%), CD40 (74%), CD80 (64%), CD83 (37%), and CD86 (96%) expression. These results are shown and quantitated in Figure 8, and are fully consistent with these cells being highly enriched for mature DCs. The infection with AAV/GM-CSF/Neo virus can fully replace the need for exogenous GM-CSF and the resulting cells appear to be a normal dendritic cell population when analyzed for a series of standard dendritic cell markers.

**The AAV/GM-CSF/Neo virus is able to chromosomally integrate in DCs.** Clearly the AAV/GM-CSF/Neo virus infection of monocytes and resulting DCs resulted in the delivery and expression of the GM-CSF and Neo genes. However, while AAV is usually known to transduce cells by chromosomal integration, there have been some who report that AAV can remain as an episomal element and thus transient element.(48,51,78) Thus, experiments were designed to study the state of the AAV proviral genome within the infected monocyte/dendritic cel precursor populations. Adherent monocytes were treated with AAV/GM-CSF/Neo virus, IL-4, and TNFα as in the FACS analysis studies in Figure 8. From this population CD83 + cells (10⁵ cells), DCs, were isolated by cell sorting. To observe chromosomal integration, PCR amplification of the AAV provirus-chromosomal DNA junction was carried out using total cellular DNA as a template which had been isolated from these sorted CD83 + DCs. The homology and strategy of these primers are shown in Figure 1B, with one primer targeting the SV40 early promoter of the provirus and the other targeting the well characterized *Alu* I repetitive element. This analysis is similar to that done by the Terwilliger laboratory to observe AAV vector integration in neurons.(65) The resulting PCR products were then separated by agarose gel electrophoresis, Southern blotted, and probed with ³²P-Neo gene probe. This final Southern blot analysis would distinguish between vector-*Alu* I products and *Alu* I*-Alu* I products. As shown in Figure 9, greater than ten ³²P-Neo positive product sizes resulted from this analysis, indicating that multiple dendritic cell clones were chromosomally transduced by the AAV/GM-CSF/Neo virus. However it is likely that the actual transduction frequency is much higher, as the AAV provirus must be located close to an *Alu* I element, and in the correct orientation for the technique to work.

Wild type AAV is known to latently infect cells at high frequency (74-76), and recombinant AAV has been shown to be a versatile virus vector, being able transduce a wide variety of cell types (43-45,48,49,57,65,66,79,80). The experiments presented herein demonstrate that AAV was able to chromosomally transduce freshly adherent monocyte/dendritic cell precursors, that the transduction of GM-CSF gene could replace the need for exogenous GM-CSF in generating DCs, and that the resulting DCs, in spite of having endogenous GM-CSF expression, displayed classical dendritic cell surface markers consistent with those displayed using the known traditional techniques (2, 3) Other viruses, including retroviruses, adenoviruses, and pox viruses, have previously been shown to transduce DCs.(12,24,29,30) This is the first report of dendritic cell transduction by AAV. Recombinant AAV stably transduces cells by chromosomal integration(43,51,65) and transduced cells are not usually a significant target of the immune system. (79-81) The data presented in this application suggests that AAV may be an effective vector for manipulating DCs.

From this data, at least five general strategies for dendritic cell genetic manipulation are considered useful and envisioned within the context of the present invention. First, the stable introduction of cytokine genes may allow for improvements in dendritic cell production or function over the exogenous addition of cytokines. As cytokine proteins have limited half-lives (t_{1/2}), benefits may be derived from continuous expression. In the future we will try the use of an AAV/GM-CSF vector along with an AAV/IL-4 vector to generate DCs. Cost benefits may also result from such a technique due to the high cost of soluble IL-4. Second, the introduction and over-expression of immunologically relevant surface proteins may cause the dendritic cell, already a potent antigen presenting cell, to be even better at this task. For example the over expession of β2 microglobulin may result in improvements of MHC class I expression and improvements in dendritic-CD8 cytotoxic T cell interaction and antigen presentation because of its role in regulating class I levels.(82-84) Third, the transduction and stable expression of the antigen gene within the dendritic cell itself may improve dendritic cell function. As with cytokines, antigen proteins degrade and are lost. The continuous expression of the antigen would prevent this loss, as compared to lipofection of the protein. Fourth, the introduction of a suicide gene, such as herpes simplex type I thymidine kinase, into the dendritic cell will allow for greater *in vivo* freedoms for using DCs as they can then be eliminated.(85) This strategy is particularly important when the antigen is an oncoprotein, such as E7's use in the immune therapy treatment of cervical cancer. Fifth, virus transduction of DCs should allow for better HLA class I presentation of the tumor and viral antigens when compared to exogenous protein introduction. Such class I presentation is critical for the induction of viral and tumor specific cytotoxic T cell response. (4)

There has been debate within the AAV research community as to whether recombinant AAV vectors are able to stably transduce hematopoietic cells by chromosomal integration, or if these vectors simply remain in an episomal form. (78) The present results are fully consistent with chromosomal integration of the AAV vector in CD83+ DCs. The variety of AAV-chromosome junction fragments that were amplified and identified also suggest that random integration of these vectors is taking place. The expansion of the G418-resistant cells shown in Figure 3 from day 15 to 18 is also consistent with stable, chromosomal transduction of the vector. Optimization for expressing genes in DCs by AAV transduction is continuing. For example, in the present application, the AAV p5 promoter was used to express the GM-CSF gene, however, other promoters that are functional in DCs can be made by selecting and utilizing promoters for their high expression in this specific cell type. The AAV/GM-CSF/Neo transduced dendritic cell population produced by AAV/GM-CSF/Neo transduction appears to express the usual markers and have the general characteristics of normal mature DCs.

**DC Pulsing Techniques:** Figure 10 provides a diagram of the structure of the experiments AAV/antigen (E6 and E7) pulsing of DCs for stimulating CTL activity against cervical cancer, and equally applicable to breast cancer, as compared to protein pulsing experiments as previously described (100).

**HPV-16 E6-specific and E7-specific CD8+ CTL Responses :** Figure 11 demonstrates that pulsing DCs with AAV-E6 results in higher CTL response when the DCs are pulsed with the AAV-E6 compared to pulsing DCs with bacterial produced antigen protein (DOTAP transfected GST-antigen protein). DC/T cell incubation was only for 7 days. The CTL activity ranged from 28-60% with the AAV/Ag vectors. It is important to note that the DC/T cell incubation in these experiments was much shorter than previous studies on only antigen protein pulsing of DCs. (1 week compared to 2-3 weeks). It was observed that the DCs died earlier when primed with the T cells earlier, when DCs were AAV-Ag pulsed compared to protein pulsing. Anti-HLA class I blocking antibody (W632) was used at 50 µg/ml. Targets were a primary cervical cancer cell line called "Mask" with a compatible haplotype to the blood donor. Figure 11 shows that killing was significantly blocked by the addition of anti-class I antibodies (W632). The lysate control is DC pulsed with lysate from 293 cells, producer cell lines.

Figure 12 shows the same experiment except AAV/E7 and GST-E7 fusion protein was used. The figure shows that the killing is much higher when AAV/E7/Neo is used to pulse the DCs.

**Analysis of T Cell Populations resulting from Different DC Pulsing:** The cell populations resulting from GST-E7/DOTAP pulsing and AAV/E7/Neo pulsing of DC were analyzed by FACS for CD56 and CD8 positivity. The results in Figure 13 show that a higher percentage of the GST-E7 pulsed cells were CD8+ compared to AAV/E7/Neo infection. Thus, if the percentage killing from CTL activity in Figure 11 is taken into account, these data strongly suggest that the increased killing by AAV/E7/Neo pulsing of DC is due to increased sensitivity/killing activity per CD8+ T cell, and not due to increased numbers of CD8+ cells.

**Onset of DC-T cell Priming Rosettes:** Figure 14 show the DC/T cell co-cultures at 2 days post-addition of T cells. Note that there are many more DC-T cell rosettes present in the AAV/E7/Neo pulsed culture than the GST-E7 and Lysate controls. This result demonstrates that priming occurs more rapidly using AAV pulsing rather than protein pulsing.

**Generation of AAV/p5-BA46/Neo, AAV/p5-Her-2/neu/Neo, and AAV/p5-CD24/Neo virus** These vectors use the AAV p5 transcriptional promoter to express the antigen genes. This promoter, although not DC-specific, is a good general purpose promoter and significantly active in many cell types. Standard recombinant DNA techniques are used. rAAV virus is produced by first generating high producer cell lines to make high-titer virus stocks. The virus stocks are CsCl-purified and titered by quantitating the amount of encapsidated genomes (virus) by Southern blot analysis. Vectors with different promoters expressing the antigen genes are performed are constructed and compared.

### Comparison of the effectiveness of the above AAV-antigen vectors with full-length BA46, Her-2/neu, and CD24 protein lipofection of DCs for their ability to stimulate responses to antigen-containing target cells

This experiment compares phenotypic characteristics (i.e.. % of CD4+, CD8+, and CD56+ cells) as well as the % cytotoxicity of BA46, Her-2, and CD24-sensitized, class I-restricted CD8+ T cells by DCs treated by AAV-Ag gene transfer and pulsed by full-length Ag protein pulsing. The DCs are generated from primary peripheral blood adherent monocytes by the standard treatment with GM-CSF and IL-4. The most important experiments are a comparison of DCs treated by antigen-gene transfer versus antigen-lipofection for their ability to stimulate CTL response against a class I restriction target. More specifically, DC pulsing by the lipofection of the full-length antigen protein (E6 or E7) and by infection with one of the following virus: AAV/BA46, AAV/Her-2, and AAV/CD24 are compared. An infection level of 1000 encapsidated genomes (EG) per cell will be used (MOI of approximately 10). Equivalent numbers of the differently treated DCs are then added to equivalent numbers of CD4+ and CD8+ T cells. After 2-3 weeks CD8+ T cell populations are tested for specific cytotoxicity against autologous tumor cells.

Either technique can be used and the selection is made by observing and comparing the number of CD8+ T cells stimulated and the percent of target cell killing in a chromium release assay using both of the DC treatments. The antigen and cytokine gene delivery protocol can be modified by changing variables. Such variables include the dosage of recombinant virus used to infect the DC, changing the time between infection and T cell incubation. Percent killing stimulated by the use of the AAV/BA46. AAV/Her-2, and AAV/CD24 virus are also compared. It is preferable to use the double virus, AAV/Ag1 +Ag2, with the highest epitope presentation potential, to provide the best in directing killing.

Another aspect of using AAV to introduce the antigen gene is to optimize the point at which to infect with the vector. The adherent monocyte is the best time at which to infect as the monocytes are still proliferating at this time point, and AAV is known to preferentially transduce dividing cells. Mature DCs do not divide and, thus, these cells may not be a good target for AAV. The comparison of immediate infection of adherent monocytes to infection of DCs after treatment with GM-CSF and IL-4 for 5-7 days is investigated.

The efficiency of vector transduction also is monitored, and specific AAV vectors that transduce DCs well are selected. Transduction is analyzed by two assays. One assay is the G418 selection/Neo resistance, as shown in Figure 4 except the cell growth is done in soft agar to allow the counting of colonies. The second assay is PCR analysis for chromosomally integrated provirus, using one primer targeting the vector and another targeting the common chromosomal repetitive element Alu I. Each PCR product (or band on the gel) is the result of one originally transformed cell. Thus, the number of products or bands provide an approximate indication of the transduction efficiency of the vector.

Adherent monocytes/DCs are isolated, treated with antigen, incubated with CD4 & 8 T cells, then CD4 and CD8 E6/E7-specific responses are measured by cytotoxic response assay against autologous tumor cells (CD8+) and proliferation by LCL lipofected with E6/E7 oncoproteins (CD4).

### Comparison of the effectiveness of the AAV/antigen vectors to the lipofection of full-length BA46, Her-2/neu, and CD24 proteins into DCs for the ability to stimulate CD4+ T cell proliferation and cytokine release

These analyses are similar to those described above. However, CD4 + helper cell proliferation and/or specific release of cytokine release are measured to show specific recognition of breast cancer antigens on antigen-presenting DCs. Adherent monocytes/dendritic cells are isolated, treated with antigen by virus (gene) or lipofection (protein), incubated with CD4+ T cells, then class II restricted proliferation or specific cytokine release (ie. INFγ or GM-CSF) measured against BA46, Her-2/neu, or CD24 positive target cells (LCL pulsed with antigen protein or infected with AAV/Ag virus). The following parameters are measured: 1) AAV/BA46 infection (1000 EG, or an MOI of approximately 10 at day 0) will be compared to full-length BA46 protein pulsing at day 5-7, 2) AAV/BA46 infection are compared at different MOIs: 100, 1000, 10,000, and 100,00 EG, 3) AAV/BA46 infection of adherent monocytes (day 0) are compared to infection of DCs at day 5-7.4) AAV/Her-2 and AAV/CD24 infection (1000 EG, MOI 10) are compared to full-length Her-2 or CD24 protein pulsing.

### Comparison of the effectiveness of AAV-BA46, AAV/Her-2/neu and AAV-CD24 to a dual antigen vector, AAV/BA46/Her-2 and AAV/Her-2/CD24 antigen vectors, for the ability to stimulate CD8+ cell cytotoxic activity

Equal numbers of DCs treated by the five techniques (single Ag vectors AAV-BA46. AAV/Her-2/neu and AAV-CD24 to a dual antigen vectors (AAV/BA46/Her-2 and AAV/Her-2/CD24) are incubated with PBMCs to induced a priming against the breast cancer antigen, as described above. After 3 weeks, a pure population of CD8+ T cells are analyzed for specific CTL response against appropriate HPV antigen-positive targets and compared for CTL response (% killing). DCs are infected with the indicated AAV/antigen virus and incubated with CD4 and CD 8 T cells. Class I restricted cytotoxicity are then measured against BA46, Her-2/neu, or CD24 positive target cells. AAV/single Ag vector infection are compared to AAV/dual Ag vector infection. Specific methods are the sdame as described above. Controls include mock-treated DCs and full-length BA46. Her-2/neu, or CD24 protein-pulsed DCs.

### Analysis of the efficiency of different class I restriction elements in BA46/Her-2/neu/CD24-derived antigen epitope presentation

It is believed that more effective immuno-stimulatory treatments are likely to result from multiple class I molecule presentation, as opposed to the dependence upon only one HLA class I restriction element. After treatment of DCs with antigen by gene delivery or protein lipofection, the dependence of the CTL response is analyzed by adding anti-class I monoclonal antibodies to block the CTL response. By using various characterized anti-class I antibodies, in conjunction with knowledge of the HLA haplotype of the individual from which the cells were derived, it is determined if the killing is dependent upon one class I molecule or if multiple class I restriction elements are involved in the presentation of BA46, Her-2/neu, or CD24- derived epitopes. Breast cancer antigen-positive targets are used to measured class I restricted CTL response in a 6-hour chromium release assay. In addition, to evaluate the HLA class I restriction elements involved in the presentation of BA46, Her-2/neu. or CD24 E7-derived epitopes, blocking antibodies specific for HLA class I A2, B7, and the monomorphic anti-class I antigens (W6/32, 50 ug/ml) (hybridomas obtained from the ATCC, Rockville, MD) are used. All assays are carried out multiple times. AAV/p5-BA46/Neo infection (1000 EG, MOI 10) are compared to full length BA46 protein pulsing for haplotype dependence. Similarly, AAV/p5-Her-2/Neo and AAV/p5-CD24/Neo infection (1000 EG, MOI 10) are compared to pulsing with those full length proteins for haplotype dependence. These analyses are performed essentially as those described above. However, anti-class I haplotypes are added to potentially block the cytotoxic activity. Any antibody which inhibits cytotoxic activity strongly suggests the involvement of that haplotype in antigen epitope presentation. Comparative controls include allogenic partially HLA A2-matched breast cancer antigen-positive and negative cell lines.

### Comparison of the efficiency of DCs transduced with AAV/BA46/Neo to DCs lipofected with BA46 protein for their ability to directly stimulate CD8+ CTL responses both with and without CD4+ T cells

Delivery of the antigen by virus infection may circumvent the need for helper CD4 + T cells. CTL activities of CD8+ T cells admixed with AAV/BA461Neo-transduced DCs, with and without CD4+ T helper cells, are compared. These analyses are carried out similarly to those described above, with the exception that pure populations of CD8 + T cells are used during priming and may or may not be supplemented with pure CD4 + T cells. Pure CD8+ T cells stimulated with AAV/p5-BA46/Neo infected DCs are analyzed for the cytotoxic activity against E7 positive target cells. Results showing significant CTL response in the absence of CD4 + T cells, are analyzed for the introduction of the antigen by AAV infection, or other factors which induce significant cytotoxic activity by CD8+ T cells stimulated with DCs. Pure CD8+ T cells stimulated with AAV/p5-BA46/Neo infected DCs are then compared to the traditional DCs lipofected with E7 protein plus CD8+T cell alone.

### Generate second-generation vectors with different transcriptional promoters (CMV, β-actin, HLA class I)

New vectors are selected by analyzing and identifying promoters which are more specific for expression in DCs and are prepared that use alternative promoters. The original vectors use the endogenous AAV p5 promoter. To identify the optimal promoter for dendritic cells, equal amounts of chloramphenicol acetyltransferase (CAT) expression plasmids are lipofected, each containing a different transcriptional promoter, into adherent monocytes. The series of CAT expression plasmids includes the AAV p5 promoter, the cytomegalovirus immediate early promoter, the β-actin promoter, and the HLA A2 class I promoter. The vectors are based on the AAV vector dI3-94, which has the p5 promoter deleted. After transfection, the cells are treated with GM-CSF and IL-4 to induce differentiation. The cells are then be analyzed for CAT activity at 2 days post-lipofection. The promoter which gives the highest level of CAT activity is used in improved, DC-optimized vectors. Standard recombinant DNA methods are used in the constructions.

### Comparison of the effectiveness of the first generation p5-driven AAV/antigen vectors to the improved vectors for their ability to effectively transduce dendritic cells and stimulate class I restricted CTL response to antigen-containing target cells

This series of experiments are very similar to the comparison of antigen delivery above, except the different AAV vectors, each with a different promoter, are compared for their ability to stimulate class I restricted CTL activity. Adherent monocytes/dendritic cells are isolated, treated with antigen, and incubated with CD4 and 8 T cells. Class I-restricted cytotoxicity are measured against target cells (HPV E6 and/or E7 positive targets). Infection of DCs with AAV/CMV-BA46/Neo, AAV/βactin-BA46/Neo, and AAV/classI-BA46/Neo (MOI 10 at day 0) are compared to the original AAV/p5-BA46/Neo vector and to full-length BA46 protein pulsing. Similarly, infection of DCs with AAV/CMV-Her-2/Neo, AAV/βactin-Her-2/Neo, and AAV/classI-Her-2/Neo (MOI 10 at day 0) are compared to the original AAV/p5-Her-2/Neo vector and to full-length Ner-2/neu protein pulsing. Methods are the same as those described above. Controls include mock-treated DCs and full-length BA46 protein-pulsed DCs.

Although the examples described above utilize the genes encoding the HPV E6 or E7 oncoproteins or the BA46, Her-2 or CD24 breast tumor markers, any gene encoding a tumors specific or disease specific antigenic protein can be substituted for these genes to prepare AAV vectors for pulsing DC precursors using the methods of the present invention.

### IMMUNOTHERAPY

Dendritic cell (DC) manipulation *in vitro* can stimulate a significant HLA class I restricted CD8+ T cell cytotoxic activity. Studies performed by several groups have recently established the key role played by DCs in the immune system and provide a rationale for using DCs as natural adjuvants for human immunotherapy. DCs are the most effective APC at activating naive T cells, and recently the combination of GM-CSF and IL-4 has been shown to generate large numbers of DCs to manipulate the immune response of autologous human T cells. The present inventors have shown that autologous DCs pulsed with full length HPV-16 or -18 E7 oncoprotein induces an HPV E7-specific T cell response from HPV-16 and -18 positive cervical cancer patients (100). Using a completely autologous system, the *in vitro* generation of E7-specific proliferative responses by autologous CD4+ lymphocytes is shown as well as the *in vitro* induction of HLA Class I-restricted CTLs against HPV-16 and -18+ naturally infected autologous cervical tumors in 3 patients with invasive cervical cancer. In addition, using two color flow cytometric analysis of intracellular cytokine expression at the single cell level, a strongly polarized type 1 pattern of cytokine secretion inducible in the E7-primed CD8 + and CD4 + populations in three patients is shown.

This is the first report of the demonstration that Class I and Class II-restricted HPV-16 and -18 E7 specific autologous lymphocytes can consistently be induced in cervical cancer patients *in vitro* using full length E7 pulsed autologous DCs. This novel approach overcomes several of the limitations imposed by the use of peptide epitope-based vaccines in an outbred population, such as humans, and therefore has important implications for the treatment of cervical cancer patients with active or adoptive immunotherapy.

Until recently there have been few reports of HPV-specific CTL responses in humans. Most of HPV-specific CTLs have been documented in mice, in which HPV is not a natural pathogen. This has lead to the suggestion that HPV has coadapted to the human host evading the immune system. However, HPV infected epithelial cells could fail to generate CTL responses, not for the lack of specific CTL precursors, but because they do not present appropriate levels of antigenic peptides in association with HLA Class I molecules, and fail to express costimulatory molecules necessary for priming of naive T cells. In agreement with this hypothesis, HPV-specfic CTLs recognizing HPV oncoproteins have recently been generated from peripheral blood of cervical cancer patients using a peptide based-epitope approach. Unfortunately, cytotoxic responses against naturally HPV infected autologous tumor cells were not tested in these studies. In this regard, previous reports have warned against the sole use of allogeneic partially HLA-matched lines or autologous in vitro pulsed/transduced cell lines as a means to reliably demonstrate specific lysis of targets endogenously expressing antigens. Indeed, CTL cell lines generated by *in vitro* primary stimulation with high concentratiion of peptides often fail to lyse targets expressing endogenous antigen.

The present inventors have demonstrated for the first time that full length E7 pulsed autologous DCs can stimulate CD8 + cytotoxic T cell response that is capable of killing autologous tumor cells in patients with invasive cervical cancer (100). It is shown that E7-pulsed DCs can elicit antigen-specific CD4 + T cell proliferative responses from the same patients. The HPV-16 E7 oncoprotein was chosen as a target antigen for the following resasons: a) HPV-16 is associated with most cervical cancers and the HPV oncogenic proteins E6 and E7 are important in the induction and maintenance of cellular transformation and are coexpressed in most HPV-containing cervical cancers, and b) E7 is a well characterized cytoplasmic/nuclear protein with little internal atypic sequence variation, and is more abundant than E6 in HPV-associated cervical cancers. This study took advantage of the cloning and expression in bacteria of E7 as a GST-fusion protein. This allowed the use of protein affinity-purification under non-denaturing conditions, and the production of large amounts of antigens for DCs pulsing. The use of the cationic lipid DOTAP was shown to be instrumental in the induction of strong and specific CD8+ CTL responses against cervical cancer tumor cells. In this regard, while the intrinsic toxicity of DOTAP on DCs was low, its function was presumably to facilitate the cytoplasmic incorporation of exogenous antigen for MHC class I restricted presentation to CD8+ T cells. Consistent with this view, it was more difficult to induce specific CTLs against autologous tumor targets than DCs pulsed overnight with E7 without DOTAP (data not shown).

Currently, epitope based immunotherapy for cervical cancer faces several important limitations. One of the major constraints is that T cell immune responses to a protein are limited to those epitopes presented by host HLA molecules. This imposes severe limitations on the use of synthetic peptides as immunogens in an heterologous (i.e. outbred) population such as human. In contrast, the use of autologous DCs pulsed with E7 of the specific HPV cancer-involved genotype leave to the autologous professional antigen presenting cells the processing and presentation of one or more epitopes in association with host HLA molecules.

Generation of potent CTL immune responses requires the presence of CD4+ helper T cells as well as the presence of both helper and CTL determinants on the same APC (150). In fact, the inability to mount a potent antitumor immune response has often been attributed to the lack of generation of sufficient tumor-specific cell help (131,132). In recent clinical studies, the *in vivo* persistence of adoptively transferred antigen specific CD8+ T cells against cytomegalovirus (133), or enhanced generation of hepatitis B specific CTLs (134) was dependent upon endogenous CD4 + responses. Moreover, the generation of tumor-reactive T helper cells have been shown to be particularly important for the immunotherapy of established tumors (ie. vascularized) and metastatic disease in several murine tumor models (135,136). Thus, the present inventors consider the stimulation of both CD8+ and CD4 + E7 specific T cell responses to be therapeutically more effective against cervical cancer than CD8 + T cells alone. It is shown that E7-pulsed DCs can readily stimulate E7-specific, HLA class II-restricted proliferatitive CD4+ T cell response. (100) Immunotherapy with both CD4 + and CD8 + T cells specific for HPV E7 may thus promote the establishment of long-term rumor specific immunosurveillance *in vivo.* T cell-mediated protection from viral infection as well as control of tumors is thought to be promoted by Type 1 cytokine responsees and impaired by Type 2 cytokine responses. In general, Type 1 T cells (CD4 or CD8) express IL-2, IFN-γ, and TNF-β, and are cytotoxic, whereas Type 2 T cells express IL-4, IL-5, IL-6, IL-10 and IL-13, provide efficient help for B cell activation, and are non-cytotoxic. Consistent with this view, IL-2 and IFN-γ producing T1 Tcells are believed to promote the development of cell mediated immunity against HPV-associated neoplasms.

Recent studies have shown significant dysfunction of Type 1 T cell responses in patients with high grade cervical intraepithelial lesions and invasive cervical cancer, suggesting that progression to cervical cancer from precursor lesions may be associated with a preferential Type 2 T cell response. (98,99) The present inventors took advantage of a recently developed flow cytometric technique for detecting intracellular cytokine expression at the single cell level in HPV-16 or -18 E7 stimulated CD8+ and CD4+ T cells (100). Two flow cytometric analysis of intracellular IFN-γ and IL-4 expression in CD8+ and CD4 + E7 specific T cells demonstrated that HPV-specific T cells from cervical cancer patients showed a major Type 1 bias in cytokine expression. Indeed, the majority of cytokine expressing T cells showed IFN-γ secretion while the minority expressed only IL-4 (Figure 4 and 5 of 100). These findings, therefore, support the view that even in patients with progressive HPV infection leading to invasive cervical cancer, presentation of HPV E7 by DCs is still able, at least *in vitro,* to activate a strong T1 response, and that Type 1 cytokine expression correlates with high cytotoxic activity against the autologous tumor cells by CD8+ T cells.

Most cancer patients do not mount an effective immune response toward their cancers. The present immunotherapeutic protocols for treating cancer are geared toward stimulating an effective cytotoxic CD8+ T cell response in the patient, and thus, generating a broad spectrum approach toward treating cancer (those with known antigen genes). The present invention takes advantage of the genetically altered DCs of the present invention containing AAV vectors containing one or more known antigen genes and/or one or more cytokines for use in stimulating the patients' T-cell response. The GM-CSF and IL4 genes are introduced into adherent monocytes by AAV vectors.

The present immunotherapy is based upon the direct delivery of one or more antigen and/or cytokine genes into DCs by an AAV vector that get stably integrated into the genome of the DCs resulting in improvements in DC production and function resulting in a superior cytotoxic CD8+ T cell response and CD4 + T cell proliferation.

The genetically altered DCs produced by the present invention are useful in an immuno-stimulatory treatment of cancer by the genetic alteration of the patient's antigen presenting DCs (DC) with full length (FL) antigen (Ag) genes. Others have shown that the introduction of a cancer Ag polypeptide into DCs by lipofection can result in a cytotoxic T cell response. The present inventors have shown that introducing FL E7 Ag into DCs with CD4 + and CD8+ T cells results in an effective class I-restricted CD8+ cytotoxic T cell response against the patients own HPV + cervical cancer cells *in vitro* (as much as 80% killing) (138). However, for all of the disadvantages discussed above regarding the degradation of proteins that are introduced exogenously, such as the introduction of antigens and cytokines via pulsing, the present invention introduces the genes that encode these antigens and cytokines via AAV vector transduction. The transduction of these genes into the chromosome of DCs provides the continuous expression of the FL antigen proteins, by AAV vector gene delivery into DCs. Virus introduction of the antigen provides an efficient way to increase class I presentation of the Ag epitopes which is critical for CD8+ cytotoxic response. Similarly, the processing and trafficking of antigen epitopes toward expression on the DC surface in the context of class I presentation is likely to be enhanced by viral delivery when compared to lipofection of the antigen protein. This form of antigen delivery to the DCs is believed to circumvent the need for CD4+ T cells for inducing final DC maturation and for the stimulation of the CD8+ T cells, resulting in a simplified regime.

The AAV vectors provide a delivery vector for the Ag and cytokine genes into DCs because of its efficiency and minimal intrinsic immunogenicity. AAV-E6, AAV-E7, and AAV-E6-E7 vector transduction efficiency is measurable and the effectiveness of antigen gene delivery is compared to traditional DC polypeptide and FL antigen protein "pulsing", by lipofection, for the ability to stimulate tumor cell killing and proliferation. Multigene AAV vectors, including HSV-TK genes (as a "suicide gene") or single AAV vectors each containing one of the genes are useful in the present invention.

Furthermore, the use of antigen oligopeptides generally limits the epitope presentation to a favored class I molecule. Thus, it is believed that using full-length antigens (FL Ags) genes or proteins result in a more generalized class I use for epitope presentation. The results of DC expression of antigens and the role of specific class I haplotype molecules on the tumor target in the class I restricted cytotoxic response are determined by specific monoclonal antibodies.

Periodically thereafter, the patient's blood is monitored for evidence of increased T-cell stimulation in the patient against the introduced disease associated antigens by methods known to persons skilled in the art. For instance, computerized tomography, x-rays and screening for tumor-related markers can be used in monitoring the success of treatment.

Although the invention has been described in detail for the purposes of illustration, it is to be understood that such detail is solely for that purpose and that variations can be made therein by those skilled in the art without departing from the spirit and scope of the invention. Particularly, the methods described herein are useful for preparing DCs that express and process any antigenic protein assoicated with a specific disease or tumor to obtain targeted DCs.

The cited references are herein incorporated in their entirety by reference.

### CITED REFERENCES

1. Steinman R.A. (1991) Annu. Rev. Imm. 9, 271-296.
2. Sallusto and Lanzavecchia, (1994) J. Exper. Med. 179(4), 1109-1118.
3. Romani, et al. (1994) J. Exper. Med. 180(1), 83-93.
4. Young and Inaba (1996) J. Exper. Med. 183, 7-11.
5. Zivotgel, et al. (1996) J. Exp. Med. 183, 87-97.
6. Paglia, et al. (1996) J. Exp. Med. 183, 317-322.
7. Alexander, et al. (1996) Am. J. Obstet. Gynecol. 175, 1586-1593.
8. Philip, et al. (1998) Cancer Gene Therapy. 5(4), 236-246.
9. Mcarthur and Mulligan (1998) J. Immunotherapy. 21(1), 41-47.
10. Fields, et al. (1998) Proc.Natl. Acad. Sci. U.S.A. 95(16), 9482-9487.
11. Sonderbye, et al. (1998) Experimental & Clinical Immunogenetics. 15(2), 100-111.
12. Kim, et al. (1997) J. Immunotherapy. 20(4), 276-286.
13. Peshwa, et al. (1998) Prostate. 36(2), 129-138.
14. Dematos, et al. (1998) J. Surgical Oncology. 74(2), 79-91.
15. Nair, et al. (1998) Nature Biotechnology. 16(4), 364-369.
16. Tjandrawan, et al. (1998) J. Immunotherapy. 21(2), 149-157.
17. Barratt-Boyes, et al. (1998) J. Immunotherapy. 21(2), 142-148.
18. Tuting, et al. (1998) J. Immunology. 160(3), 1139-1147.
19. De Bruijn, et al (1998) Cancer Research. 58(4), 724-731.
20. Tsai, et al. (1998) Critical Reviews in Immunology. 18(1-2), 65-75.
21. Wong, C., et al. (1998) J. Immunotherapy. 21(1), 32-40.
22. Alters, et al. (1998) J. Immunotherapy. 21(1), 17-26.
23. Navabi, et al. (1997) Advances in Experimental Medicine & Biology. 417, 583-589.
24. Specht, et al. (1997) J. Exper. Med. 186(8), 1213-1221.
25. Toujas, et al. (1997) Immunology. 91(4), 635-642.
26. Brossart, et al. (1997) J. Immunology. 158(7), 3270-3276.
27. Tsai, et al. (1997) J. Immunology. 158(4), 1796-1802.
28. Hermans, et al. (1997) Cancer Immunology, Immunotherapy. 44(6), 341-347.
29. Reeves, et al. (1996) Cancer Research. 56(24), 5672-5677.
30. Smith, et al. (1996) Journal of Virology. 70(10), 6733-6740.
31. Van Elsas, et al. (1996) European Journal of Immunology. 26(8), 1683-1689.
32. Bianchi, et al. (1996) Journal of Immunology. 157(4), 1589-1597.
33. Mjaaland, And Fossum, (1995) Scandinavian Journal of Immunology. 41(3), 305-308.
34. Bei, et al. (1998) Journal of Immunotherapy. 21(3), 159-169.
35. Nair, et al. (1992) Journal of Immunological Methods. 152(2), 237-243.
36. Fawell, et al. (1994) Proc. Natl. Acad. Sci. U.S.A. 91(2), 664-668.
37. Graziadei, et al. (1991) Analytical Biochemistry. 194(1), 198-203.
38. Kim, et al. (1991) Experimental Cell Research. 197(2), 207-212.
39. Sugawa, et al. (1985) Experimental Cell Research. 159(2), 419-429.
40. Ortiz, et al. (1987) Molec. Cell. Biol. 7, 3012-3017.
41. Kedar, et al. (1997) Journal of Immunotherapy. 20(3), 180-193.
42. Conlon, et al. (1989) Biotechnology Therapeutics. 1(1). 31-41.
43. Hermonat and Muzyczka (1984) Proc. Natl. Acad. Sci. U.S.A. 81, 6466-6470.
44. Laface, et al. (1988) Virology 162, 483-486.
45. Zhou, et al. (1993) Experimental Hematology. 21(7), 928-933.
46. Zhou, et al. (1994) Journal of Experimental Medicine. 179(6), 1867-1875.
47. Goodman, et al. (1994) Blood. 84(5), 1492-1500.
48. Podsakoff, et al. (1994) Journal of Virology. 74(9), 5656-5666.
49. Chiorini, et al. (1995) Human Gene Therapy. 6(12), 1531-1541.
50. Lubovy, et al. (1996) Biology of Blood & Marrow Transplantation. 2(1), 24-30.
51. Fisher-Adams, et al. (1996) Blood. 88(2), 492-504.
52. Ponnazhagan, et al. (1997) Journal of Virology. 71(11), 8262-8267.
53. Ponnazhagan, et al. (1997) Journal of Virology. 71(4), 3098-3104.
54. Samulski, et al. (1982) Proc. Natl. Acad. Sci. U.S.A. 79(6), 2077-2081.
55. Russell, et al. (1994) Proc.Natl. Acad. Sci. U.S.A. 91(19), 8915-8919.
56. Zolotukhin, et al. (1996) Journal of Virology. 70(7), 4646-5464.
57. Hermonat, et al. (1997) FEBS Letters 407, 78-84.
58. Hirt, B. (1967) Journal of Molecular Biology. 26(2), 365-370.
59. Southern, E.M. (1975) Journal of Molecular Biology. 98(3), 503-517.
60. Hermonat, et al. (1997) Virus Genes. 14(1), 13-17.
61. Pisa, et al. Proc.Natl. Acad. Sci. U.S.A. 89(16), 7708-7712.
62. Batzer, et al. (1991) Nucleic Acids Research. 19(13), 3619-1322.
63. Southern And Berg (1982) Journal of Molecular & Applied Genetics. 1(4), 327-340.
64. Santin, et al. (1999) Immunobiology 200, 187-204.
65. Wu. et al. (1998) Journal of Virology. 72(7), 5919-5927.
66. Alexander, et al. (1996) Human Gene Therapy. 7(7), 841-850.
67. Russell, et al. (1995) Proc. Natl. Acad. Sci. U.S.A. 92(12), 5719-5723.
68. Halbert, et al. (1995) Journal of Virology. 69(3), 1473-1479.
69. Wright-Browne, et al. (1997) Human Pathology. 28(5), 563-579.
70. Macpherson, et al. (1995) Journal of Immunology. 154(3), 1317-1723.
71. Cumberbatch And Kimber (1995) Immunology. 84(1), 31.
72. Szabolcs, et al. (1995) Journal of Immunology. 154(11), 5851.
73. Lardon, et al. (1997) Immunology. 91(4), 553-559.
74. Hoggan, M.D., Thomas And Johnson (1972) Proceedings of the fourh lepetite colloquium. Cocoyac, Mexico. North-Holland, Amsterdam., pp243-249.
75. Cheung, A.K.M., et al. (1980) Journal of Virology. 33, 739-748.
76. Laughlin, et al. (1986) Journal of Virology 60, 515-524.
77. Fisher, et al. (1997) Nature Medicine. 3(3), 306-312.
78. Malik et al. (1998) Journal of Virology. 71, 1776-1783.
79. Monahan, et al. (1998) Gene Therapy. 5(1), 40-49.
80. Clark, et al. (1997) Human Gene Therapy. 8(6), 659-669.
81. Xiao And Samulski (1996) Journal of Virology. 70(11), 8098-8108.
82. Pook, et al. (1991) Human Immunology. 32(2), 102-109.
83. Hicklin, et al. (1998) Journal of Clinical Investigation. 101(12), 2720-2729.
84. Hicklin, et al. (1997) Melanoma Research. 7 Suppl 2:S67.
85. Tiberghien, et al. (1994) Blood. 84(4), 1333-1341.
86. Hermonat, et al. (1984) J. Virology 51(2):329-339.
87. Tratschin, et al. (1984) J. Virol. 51:611-619.
88. Tratschin, et al. (1985) Mol. Cell. Biol. 5:3251-3260.
89. Kotin, et al. (1990) Poc. Natl. Acad. Sci. U.S.A. 87:2211-2215.
90. Samulski, et al. (1991) EMBO J. 1991; 10:3941-50.
91. Kotin, et al. (1992) EMBO J. 11:5071-5078
92. McLaughlin SK, et al. J. Virol. 1988; 62:1963-1973.
93. Lebkowski JS, et al. (1988) Mol. Cell. Biol. 8:3988-3996.
94. Samulski RJ, et al. (1989) J. Virol. 63:3822-3828.
95. Chatterjee S, et al. (1995) Ann. N.Y. Acad. Sci. 770:79-90
96. Flotte TR, et al. (1995) Gene Therapy 2:29-37.
97. Urcelay, et al. (1995) J. Virol. 69:2038-2046.
98. Tsukui, et al. (1996) Cancer Research, 56:3967-3974.
99. Clerici, et al. (1997) J. Natl. Cancer Instit. 264: 961-965.
100. Santin et al. (1999) J.Virology 73(7): 5402-5410.
101. Ionnides And Whiteside (1993) Clin. Immunol. Immunopathol. 66:91-106.
102. Gabrilovich et al. (1997) Clinical Cancer Research. 3(3):483-90.
103. Giuffrida et at. (1998) Journal of Protein Chemistry. 17(2):143-8.
104. Larocca et al. (1991) Cancer Research. 51(18):4994-8.
105. Couto et al. (1996) DNA & Cell Biology. 15(4):281-6.
106. Taylor et al. (1997) DNA & Cell Biology. 16(7):861-869.
107. Aoki et al. (1995) Biochimica et Biophysica Acta. 1245(3):385-391.
108. Ceriani et al. (1995) Cancer Research. 55(23 Suppl):5852s-5856s.
109. Peterson, et al. (1995) Cancer Research. 55(23 Suppl):5847s-5851s.
110. Couto, et al. (1995) Cancer Research. 55(8):1717-1722.
111. Beug, et al. (1980) Proc. Natl. Acad. Sci. USA. 77(11):6683-6.
112. Sergeant, et al. (1982) EMBO Journal. 1(2):237-242.
113. Bacus, et al. (1990) Archives of Pathology & Laboratory Medicine. 114(2):164-169.
114. Natali, et al. (1990) International Journal of Cancer. 45(3):457-461.
115. Slamon, et al. (1987) Science. 235(4785):177-182.
116. Van de Vijver (1987) Molecular & Cellular Biology. 7(5):2019-2023
117. Fendly, et al. (1990) Journal of Biological Response Modifiers. 9(5):449-455
118. Priuccello, et al. (1985) J. Immunology 136:3779-3784
119. Fischer, et al. (1990) J. Immunology 144:638-641.
120. Kay, et al. (1991) J. Immunology 147:1412-1416.
121. Jackson, et al. (1992) Cancer Research 51:5264-5270.
122. Akashi, et al. (1994) Virchows Arch. 425:399-406.
123. Fogel, et al. (1999) Cancer Letters. 143(1):87-94.
124. List of CD molecules, prow@ncbi.nlm.nih.gov
125. Cefai et al. (1999) Internat. J. Cancer. 83(3):393-400.
126. Peptide et al. (1999) Anticancer Research. 19(4A):2471-5
127. Kawashima et al. (1999) Cancer Research. 59(2):431-435
128. Amici, et al. (1998) Cancer Immunology, Immunotherapy. 47(4):183-190
129. Zaks and Rosenberg (1998) Cancer Research. 58(21):4902-4908
130. Bennet, et al. (1997) J. Exp. Med. 186: 65-70.
131. Baskar, et al. (1994) Cell. Immunol. 155:123-133
132. Ossendorp, et al.(1998) 1. Exp. Med. 187: 693-702.
133. Walker, et al.(1995) N. Engl. J. Med. 333: 1038-1044.
134. Vitiello, et al. (1995) J. Clinical. Investig. 95: 341-349.
135. Baskar, et al.(1998) J. Exp. Med. 181: 619-629.
136. Pulaski and Ostrand-Rosenberg (1998) Cancer Res. 58: 1486-1493.
137. Von Herrath, et al. (1996) J. Virology 70: 1072-1079.
138. Romagnani, S. (1992) Intern. Arch. Allergy Immunology. 98:279-285.
139. Liu et al. (2000) J. Interferon and Cytokine Research 20:21-30.
140. Dias et al. (1998) International J. of Cancer 75 (1): 151-157.

## Claims

1. A method for preparing genetically altered dendritic cells, wherein said method comprises:
(a) preparing adherent dendritic cell precursors from dendritic cell precursors obtained from a human subject; and
(b) transducing said adherent dendritic cell precursors with at least one recombinant adeno-associated virus (AAV) comprising at least one heterologous gene to obtain genetically altered dendritic cells expressing said heterologous gene.

2. A method for preparing genetically altered dendritic cells, wherein said method comprises:
(a) preparing adherent dendritic cell precursors from dendritic cell precursors obtained from a human subject;
(b) transducing said adherent dendritic cell precursors with at least one recombinant adeno-associated virus (AAV) comprising at least one heterologous gene selected from the group consisting of a gene encoding an antigen, a gene encoding a cytokine and combinations of one or more genes thereof; and
(c) incubating said adherent dendritic cell precursors in medium under conditions whereby said adherent dendritic cell precursors differentiate into dendritic cells that contain said heterologous gene stably integrated into the genome of said dendritic cell to obtain genetically altered dendritic cells.

3. The method of claim 1 or 2, wherein said heterologous gene encodes at least one cytokine that promotes cell proliferation of said transduced precursors to produce genetically altered dendritic cells.

4. The method of any one of claims 1 to 3, wherein said method further comprises incubating said transduced precursors in medium, and subsequently adding at least one cytokine that promotes cell proliferation of said transduced precursors after transduction.

5. The method of claim 4, wherein said medium contains at least one cytokine that promotes cell proliferation of said transduced precursors.

6. The method of claim 2, wherein said medium comprises at least one cytokine that promotes cell proliferation of said precursors and wherein a cytokine that promotes differentiation of said precursors is subsequently added to said medium after transduction.

7. The method of any one of claims 4 to 6, wherein said cytokine that promotes cell proliferation is GM-CSF and said cytokine that promotes differentiation of said precursors is IL-4.

8. The method of any one of claims 2 to 7, wherein said gene encodes at least one cytokine selected from the group consisting of CSF, interleukin (IL), tumor necrosis factor (TNF) and combinations thereof.

9. The method of any one of claims 2 to 8, wherein said antigen is selected from the group consisting of a disease specific antigen, a pathogen specific antigen or a tumor specific antigen.

10. The method of claim 9, wherein said antigen is selected from the group consisting of human papilloma virus antigen, a hepatitis antigen, a HIV antigen and a breast cancer specific antigen.

11. The method of claim 9 or 10, wherein said antigen is selected from the group consisting of human papilloma virus E6, human papilloma virus E7, human papilloma virus L1 capsid gene, a hepatitis B virus antigen, a hepatitis C virus antigen, BA46, Her-2/neu and CD24.

12. A genetically altered dendritic cell produced by the method of any one of claims 1 to 11.

13. Use of genetically altered dendritic cells comprising at least one recombinant AAV comprising at least one heterologous gene selected from the group consisting of a gene encoding an antigen associated with a disease, a gene encoding a cytokine and combinations of one or more genes thereof for the preparation of a pharmaceutical composition for stimulating the immune system of a patient afflicted with said disease, whereby said heterologous gene is expressed in said dendritic cells and said patient's immune system is stimulated by the stimulation of said patient's T cell response.

14. Use of claim 13, wherein said genetically altered dendritic cells are obtained according to a method comprising:
(a) preparing adherent dendritic cell precursors from dendritic cell precursors obtained from said patient;
(b) transducing said adherent dendritic cell precursors with at least one recombinant AAV comprising at least one heterologous gene selected from the group consisting of a gene encoding an antigen associated with said disease, a gene encoding a cytokine and combinations of one or more genes thereof; and
(c) incubating said adherent dendritic cell precursors in medium under conditions whereby said adherent dendritic cell precursors differentiate into dendritic cells that contain said heterologous gene stably integrated into the genome of said dendritic cell to obtain genetically altered dendritic cell.

15. Use of claim 13 or 14, wherein said heterologous gene encodes at least one cytokine that promotes cell proliferation of said transduced precursors to produce genetically altered dendritic cells.

16. Use of claim 14, wherein said method further comprises incubating said transduced precursors in medium, and subsequently adding at least one cytokine that promotes differentiation of said transduced precursors after transduction.

17. Use of claim 16, wherein said medium contains at least one cytokine that promotes cell proliferation of said transduced precursors.

18. Use of claim 14, wherein said medium comprises at least one cytokine that promotes cell proliferation of said precursors and wherein a cytokine that promotes differentiation of said precursors is subsequently added to said medium after transduction.

19. Use of any one of claims 15 to 18, wherein said cytokine that promotes cell proliferation is GM-CSF and said cytokine that promotes differentiation of said precursors is IL-4.

20. Use of any one of claims 13 to 19, wherein said gene encodes at least one cytokine selected from the group consisting of CSF, interleukin (IL), tumor necrosis factor (TNF) and combinations thereof.

21. Use of any one of claims 13 to 20, wherein said antigen is selected from the group consisting of a disease specific antigen, a pathogen specific antigen or a tumor specific antigen.

22. Use of claim 21, wherein said antigen is selected from the group consisting of human papilloma virus antigen, a hepatitis antigen, a HIV antigen and a breast cancer specific antigen.

23. Use of claim 22, wherein said antigen is selected from the group consisting of human papilloma virus E6, human papilloma virus E7, human papilloma virus L1 capsid gene, a hepatitis B virus antigen, a hepatitis C virus antigen, BA46, Her-2/neu and CD24.

24. The use of any one of claims 14 to 23, wherein said method further comprises incubating said genetically altered dendritic cells with said patient's CD8+ cytotoxic T cells priors to administering said genetically altered dendritic cells to said patient.

25. The use of any one of claims 14 to 24, wherein said method comprises incubating said genetically altered dendritic cells with said patient's CD4+ helper T cells to obtain stimulated T cells.

26. Use of genetically altered dendritic cells of claim 12 for the manufacture of a medicament for the stimulation of the immune response of a patient afflicted with a disease.

27. A pharmaceutical composition comprising a pharmaceutically acceptable carrier and genetically altered dendritic cells comprising a recombinant AAV comprising at least one heterologous gene selected from the group consisting of a gene encoding an antigen, a gene encoding a cytokine and combinations of one or more genes thereof stably integrated into the genome of said dendritic cell.

28. The pharmaceutical composition of claim 27, wherein said genetically altered dendritic cells are obtained by a method comprising
(a) preparing adherent dendritic cell precursors from dendritic cell precursors obtained from a human subject; and
(b) transducing said adherent dendritic cell precursors with at least one recombinant AAV comprising at least one heterologous gene to obtain genetically altered dendritic cells expressing said heterologous gene; and
(c) incubating said adherent cell precursors in medium under conditions whereby said adherent dendritic cell precursors differentiate into dendritic cells that contain said heterologous gene stably integrated into the genome of said dendritic cell to obtain genetically altered dendritic cells.

## Patentansprüche

1. Verfahren zur Herstellung genetisch veränderter dendritischer Zellen, wobei das Verfahren umfasst:
(a) Bereitstellen von adhärenten dendritischen Zellvorläufern aus von einem Menschen erhaltenen dendritischen Zellvorläufern; und
(b) Transduzieren der adhärenten dendritschen Zellvorläufer mit mindestens einem rekombinanten adenoassozüerten Virus (AAV) umfassend mindestens ein heterologes Gen, um genetisch veränderte dendritsche Zellen zu erhalten, die das heterologe Gen exprimieren.

2. Verfahren zur Herstellung von genetisch veränderten dendritischen Zellen, wobei das Verfahren umfasst:
(a) Bereitstellen von adhärenten dendritischen Zellvorläufern aus von einem Menschen erhaltenen dendritschen Zellvorläufern;
(b) Transduzieren der adhärenten dendritischen Zellvorläufer mit mindestens einem rekombinanten adenoassozüerten Virus (AAV) umfassend mindestens ein heterologes Gen ausgewählt aus der Gruppe bestehend aus einem für ein Antigen kodierenden Gen, einem für ein Zytokin kodierenden Gen und Kombinationen von einem oder mehreren dieser Gene; und
(c) Inkubieren der adhärenten dendritischen Zellvorläufer in Medium unter Bedingungen, durch die die adhärenten dendritischen Zellvorläufer in dendritische Zellen differenzieren, die das heterologe Gen stabil in das Genom der dendritschen Zelle integriert enthalten, um genetisch veränderte dendritsche Zellen zu erhalten.

3. Verfahren nach Anspruch 1 oder 2, wobei das heterologe Gen mindestens ein Zytokin kodiert, das die Zellproliferation der transduzierten Vorläufer fördert, um genetisch veränderte dendritische Zellen herzustellen.

4. Verfahren nach einem der Ansprüche 1 bis 3, wobei das Verfahren weiter umfasst: das Inkubieren der transduzierten Vorläufer in Medium und im Anschluss an die Transduktion das Hinzufügen mindestens eines Zytokins, das die Zellproliferation der transduzierten Vorläufer fördert.

5. Verfahren nach Anspruch 4, wobei das Medium mindestens ein Zytokin enthält, das die Zellproliferation der transduzierten Vorläufer fördert.

6. Verfahren nach Anspruch 2, wobei das Medium mindestens ein Zytokin enthält, das die Zellproliferation der Vorläufer fördert, und wobei ein Zytokin, das die Differenzierung der Vorläufer fördert, im Anschluss an die Transduktion zum Medium hinzugefügt wird.

7. Verfahren nach einem der Ansprüche 4 bis 6, wobei das die Zellproliferation fördernde Zytokin GM-CSF ist und das die Differenzierung der Vorläufer fördernde Zytokin IL-4 ist.

8. Verfahren nach einem der Ansprüche 2 bis 7, wobei das Gen mindestens ein Zytokin ausgewählt aus der Gruppe bestehend aus CSF, Interleukin (IL), Tumornekrosefaktor (TNF) und Kombinationen daraus kodiert.

9. Verfahren nach einem der Ansprüche 2 bis 8, wobei das Antigen ausgewählt ist aus der Gruppe bestehend aus einem krankheitsspezifischen Antigen, einem pathogenspezifischen Antigen oder einem tumorspezifischen Antigen.

10. Verfahren nach Anspruch 9, wobei das Antigen ausgewählt ist aus der Gruppe bestehend aus humanem Papillomavirusantigen, einem Hepatitisantigen, einem HIV-Antigen und einem Brustkrebs-spezifischen Antigen.

11. Verfahren nach Anspruch 9 oder 10, wobei das Antigen ausgewählt ist aus der Gruppe bestehend aus humanem Papillomavirus E6, humanem Papillomavirus E7, humanem Papillomavirus L1-Kapsidgen, einem Hepatitis B-Virus-Antigen, einem Hepatitis C-Virus-Antigen, BA46, Her-2/neu und CD24.

12. Genetisch veränderte dendritsche Zelle, hergestellt durch das Verfahren nach einem der Ansprüche 1 bis 11.

13. Verwendung genetisch veränderter dendritscher Zellen, umfassend mindestens ein rekombinantes AAV umfassend mindestens ein heterologes Gen ausgewählt aus der Gruppe bestehend aus einem Gen, das ein mit einer Krankheit assoziierte Antigen kodiert, einem Gen, das ein Zytokin kodiert, und Kombinationen von einem oder mehreren dieser Gene für die Herstellung einer pharmazeutischen Zusammensetzung zur Stimulation des Immunsystems eines Patienten, der von der Krankheit betroffen ist, wobei das heterologe Gen in den dendritischen Zellen exprimiert wird und das Immunsystem des Patienten durch die Stimulation der T-Zellantwort des Patienten stimuliert wird.

14. Verwendung nach Anspruch 13, wobei die genetisch veränderten dendritischen Zellen durch ein Verfahren erhalten werden, das umfasst:
(a) Bereitstellen von adhärenten dendritischen Zellvorläufern aus vom Patienten erhaltenen dendritschen Zellvorläufern;
(b) Transduzieren der adhärenten dendritischen Zellvorläufer mit mindestens einem rekombinanten AAV umfassend mindestens ein heterologes Gen ausgewählt aus der Gruppe bestehend aus einem für ein mit der Krankheit assoziierten Antigen kodierenden Gen, einem für ein Zytokin kodierenden Gen und Kombinationen von einem oder mehreren dieser Gene; und
(c) Inkubieren der adhärenten dendritischen Zellvorläufer in Medium unter Bedingungen, durch die die adhärenten dendritischen Zellvorläufer in dendritische Zellen differenzieren, die das heterologe Gen stabil in das Genom der dendritschen Zelle integriert enthalten, um genetisch veränderte dendritsche Zellen zu erhalten.

15. Verwendung nach Anspruch 13 oder 14, wobei das heterologe Gen mindestens ein Zytokin kodiert, das die Zellproliferation der transduzierten Vorläufer fördert, um genetisch veränderte dendritische Zellen zu produzieren.

16. Verwendung nach Anspruch 14, wobei das Verfahren weiter umfasst: das Inkubieren der transduzierten Vorläufer in Medium und im Anschluss an die Transduktion das Hinzufügen mindestens eines Zytokins, das die Differenzierung der transduzierten Vorläufer fördert.

17. Verwendung nach Anspruch 16, wobei das Medium mindestens ein Zytokin enthält, das die Zellproliferation der transduzierten Vorläufer fördert.

18. Verwendung nach Anspruch 14, wobei das Medium mindestens ein Zytokin enthält, das die Zellproliferation der Vorläufer fördert, und wobei ein Zytokin, das die Differenzierung der Vorläufer fördert, im Anschluss an die Transduktion zum Medium hinzugefügt wird.

19. Verwendung nach einem der Ansprüche 15 bis 18, wobei das die Zellproliferation fördernde Zytokin GM-CSF ist und das die Differenzierung der Vorläufer fördernde Zytokin IL-4 ist.

20. Verwendung nach einem der Ansprüche 13 bis 19, wobei das Gen mindestens ein Zytokin ausgewählt aus der Gruppe bestehend aus CSF, Interleukin (IL), Tumornekrosefaktor (TNF) und Kombinationen daraus kodiert.

21. Verwendung nach einem der Ansprüche 13 bis 20, wobei das Antigen ausgewählt ist aus der Gruppe bestehend aus einem krankheitsspezifischen Antigen, einem pathogenspezifischen Antigen oder einem tumorspezifischen Antigen.

22. Verwendung nach Anspruch 21, wobei das Antigen ausgewählt ist aus der Gruppe bestehend aus humanem Papillomavirusantigen, einem Antigen.

23. Verwendung nach Anspruch 22, wobei das Antigen ausgewählt ist aus der Gruppe bestehend aus humanem Papillomavirus E6, humanem Papillomavirus E7, humanem Papillomavirus L1-Kapsidgen, einem Hepatitis B-Virus-Antigen, einem Hepatitis C-Virus-Antigen, BA46, Her-2/neu und CD24.

24. Verwendung nach einem der Ansprüche 14 bis 23, wobei das Verfahren weiter umfasst: das Inkubieren der genetisch veränderten dendritischen Zellen mit den CD8+-zytotoxischen T-Zellen des Patienten vor Verabreichung der genetisch veränderten dendritischen Zellen an den Patienten.

25. Verwendung nach einem der Ansprüche 14 bis 24, wobei das Verfahren weiter umfasst: das Inkubieren der genetisch veränderten dendritischen Zellen mit den CD4+ T-Helferzellen des Patienten, um stimulierte T-Zellen zu erhalten.

26. Verwendung der genetisch veränderten dendritischen Zellen nach Anspruch 12 für die Herstellung eines Medikamentes zur Stimulation der Immunantwort eines von einer Krankheit betroffenen Patienten.

27. Pharmazeutische Zusammensetzung umfassend einen pharmazeutisch verträglichen Träger und genetisch veränderte dendritische Zellen umfassend ein rekombinantes AAV umfassend mindestens ein heterologes Gen ausgewählt aus der Gruppe bestehend aus einem für ein Antigen kodierenden Gen, einem für ein Zytokin kodierenden Gen und Kombinationen von einem oder mehreren dieser Gene, die stabil in das Genom der dendritischen Zellen integriert sind.

28. Pharmazeutische Zusammensetzung nach Anspruch 27, wobei die genetisch veränderten dendritischen Zellen durch ein Verfahren erhalten werden, das umfasst:
(a) Bereitstellen von adhärenten dendritischen Zellvorläufern aus von einem Menschen erhaltenen dendritischen Zellvorläufern; und
umfasst:
(a) Bereitstellen von adhärenten dendritischen Zellvorläufern aus von einem Menschen erhaltenen dendritischen Zellvorläufern; und
(b) Transduzieren der adhärenten dendritischen Zellvorläufer mit mindestens einem rekombinanten AAV umfassend mindestens ein heterologes Gen, um genetisch veränderte dendritische Zellen zu erhalten, die das heterologe Gen exprimieren; und
(c) Inkubieren der adhärenten dendritischen Zellvorläufer in Medium unter Bedingungen, durch die die adhärenten dendritischen Zellvorläufer in dendritische Zellen differenzieren, die das heterologe Gen stabil in das Genom der dendritschen Zelle integriert enthalten, um genetisch veränderte dendritsche Zellen zu erhalten.

## Revendications

1. Procédé de préparation de cellules dendritiques génétiquement modifiées, dans lequel ledit procédé comprend :
(a) la préparation de précurseurs de cellules dendritiques adhérents à partir de précurseurs de cellules dendritiques obtenus auprès d'un sujet humain ; et
(b) la transduction desdits précurseurs de cellules dendritiques adhérents avec au moins un virus adéno-associé (AAV) recombinant comprenant au moins un gène hétérologue pour obtenir des cellules dendritiques génétiquement modifiées exprimant ledit gène hétérologue.

2. Procédé de préparation de cellules dendritiques génétiquement modifiées, dans lequel ledit procédé comprend :
(a) la préparation de précurseurs de cellules dendritiques adhérents à partir de précurseurs de cellules dendritiques obtenus auprès d'un sujet humain ;
(b) la transduction desdits précurseurs de cellules dendritiques adhérents avec au moins un virus adéno-associé (AAV) recombinant comprenant au moins un gène hétérologue choisi dans le groupe constitué d'un gène codant un antigène, d'un gène codant une cytokine et de combinaisons d'un ou plusieurs gènes de ceux-ci ; et
(c) l'incubation desdits précurseurs de cellules dendritiques adhérents dans un milieu dans des conditions où lesdits précurseurs de cellules dendritiques adhérents se différencient en des cellules dendritiques qui contiennent ledit gène hétérologue intégré de manière stable dans le génome de ladite cellule dendritique pour obtenir des cellules dendritiques génétiquement modifiées.

3. Procédé selon la revendication 1 ou 2, dans lequel ledit gène hétérologue code au moins une cytokine qui favorise la prolifération cellulaire desdits précurseurs ayant subi une transduction pour produire des cellules dendritiques génétiquement modifiées.

4. Procédé selon l'une quelconque des revendications 1 à 3, dans lequel ledit procédé comprend en outre l'incubation desdits précurseurs ayant subi une transduction dans un milieu, et l'addition ultérieure d'au moins une cytokine qui favorise la prolifération cellulaire desdits précurseurs ayant subi une transduction après la transduction.

5. Procédé selon la revendication 4, dans lequel ledit milieu contient au moins une cytokine qui favorise la prolifération cellulaire desdits précurseurs ayant subi une transduction.

6. Procédé selon la revendication 2, dans lequel ledit milieu comprend au moins une cytokine qui favorise la prolifération cellulaire desdits précurseurs et dans lequel une cytokine favorisant la différenciation desdits précurseurs est ultérieurement ajoutée audit milieu après transduction.

7. Procédé selon l'une quelconque des revendications 4 à 6, dans lequel ladite cytokine qui favorise la prolifération cellulaire est le GM-CSF et ladite cytokine qui favorise la différenciation desdits précurseurs est l'IL-4.

8. Procédé selon l'une quelconque des revendications 2 à 7, dans lequel ledit gène code au moins une cytokine choisie dans le groupe constitué du CSF, de l'interleukine (IL), du facteur de nécrose tumorale (TNF) et de leurs combinaisons.

9. Procédé selon l'une quelconque des revendications 2 à 8, dans lequel ledit antigène est choisi dans le groupe constitué d'un antigène spécifique d'une maladie, d'un antigène spécifique d'un pathogène ou d'un antigène spécifique d'une tumeur.

10. Procédé selon la revendication 9, dans lequel ledit antigène est choisi dans le groupe constitué de l'antigène du papillomavirus humain, d'un antigène de l'hépatite, d'un antigène du VIH et d'un antigène spécifique au cancer du sein.

11. Procédé selon la revendication 9 ou 10, dans lequel ledit antigène est choisi dans le groupe constitué du papillomavirus humain E6, du papillomavirus humain E7, du gène de capside du papillomavirus humain L1, d'un antigène du virus de l'hépatite B, d'un antigène du virus de l'hépatite C, de BA46, de Her-2/neu et de CD24.

12. Cellule dendritique génétiquement modifiée produite par le procédé selon l'une quelconque des revendications 1 à 11.

13. Utilisation de cellules dendritiques génétiquement modifiées comprenant au moins un AAV recombinant comprenant au moins un gène hétérologue choisi dans le groupe constitué d'un gène codant un antigène associé à une maladie, d'un gène codant une cytokine et de combinaisons d'un ou plusieurs gènes de ceux-ci pour la préparation d'une composition pharmaceutique pour stimuler le système immunitaire d'un patient souffrant de ladite maladie, où ledit gène hétérologue est exprimé dans lesdites cellules dendritiques et ledit système immunitaire du patient est stimulé par la stimulation de ladite réponse des lymphocytes T du patient.

14. Utilisation selon la revendication 13, dans laquelle lesdites cellules dendritiques génétiquement modifiées sont obtenues selon un procédé comprenant :
(a) la préparation de précurseurs de cellules dendritiques adhérents à partir de précurseurs de cellules dendritiques obtenus auprès dudit patient ;
(b) la transduction desdits précurseurs de cellules dendritiques adhérents avec au moins un AAV recombinant comprenant au moins un gène hétérologue choisi dans le groupe constitué d'un gène codant un antigène associé à ladite maladie, d'un gène codant une cytokine et de combinaisons d'un ou plusieurs gènes de ceux-ci ; et
(c) l'incubation desdits précurseurs de cellules dendritiques adhérents dans un milieu dans des conditions où lesdits précurseurs de cellules dendritiques adhérents se différencient en des cellules dendritiques qui contiennent ledit gène hétérologue intégré de manière stable dans le génome de ladite cellule dendritique pour obtenir une cellule dendritique génétiquement modifiée.

15. Utilisation selon la revendication 13 ou 14, dans laquelle ledit gène hétérologue code au moins une cytokine qui favorise la prolifération cellulaire desdits précurseurs ayant subi une transduction pour produire des cellules dendritiques génétiquement modifiées.

16. Utilisation selon la revendication 14, dans laquelle ledit procédé comprend en outre l'incubation desdits précurseurs ayant subi une transduction dans un milieu, et l'addition ultérieure d'au moins une cytokine qui favorise la différenciation desdits précurseurs ayant subi une transduction après la transduction.

17. Utilisation selon la revendication 16, dans laquelle ledit milieu contient au moins une cytokine qui favorise la prolifération cellulaire desdits précurseurs ayant subi une transduction.

18. Utilisation selon la revendication 14, dans laquelle ledit milieu comprend au moins une cytokine qui favorise la prolifération cellulaire desdits précurseurs et dans laquelle une cytokine qui favorise la différenciation desdits précurseurs est ajoutée ultérieurement audit milieu après transduction.

19. Utilisation selon l'une quelconque des revendications 15 à 18, dans laquelle ladite cytokine qui favorise la prolifération cellulaire est le GM-CSF et ladite cytokine qui favorise la différenciation desdits précurseurs est l'IL-4.

20. Utilisation selon l'une quelconque des revendications 13 à 19, dans laquelle ledit gène code au moins une cytokine choisie dans le groupe constituée du CSF, de l'interleukine (IL), du facteur de nécrose tumorale (TNF) et de leurs combinaisons.

21. Utilisation selon l'une quelconque des revendications 13 à 20, dans laquelle ledit antigène est choisi dans le groupe constitué d'un antigène spécifique d'une maladie, d'un antigène spécifique d'un pathogène ou d'un antigène spécifique d'une tumeur.

22. Utilisation selon la revendication 21, dans laquelle ledit antigène est choisi dans le groupe constitué de l'antigène du papillomavirus humain, d'un antigène de l'hépatite, d'un antigène du VIH et d'un antigène spécifique au cancer du sein.

23. Utilisation selon la revendication 22, dans laquelle ledit antigène est choisi dans le groupe constitué du papillomavirus humain E6, du papillomavirus humain E7, du gène de capside du papillomavirus humain L1, d'un antigène du virus de l'hépatite B, d'un antigène du virus de l'hépatite C, de BA46, de Her-2/neu et de CD24.

24. Utilisation selon l'une quelconque des revendications 14 à 23, le procédé comprenant en outre l'incubation desdites cellules dendritiques génétiquement modifiées avec les lymphocytes T cytotoxiques CD8+ dudit patient avant l'administration desdites cellules dendritiques génétiquement modifiées audit patient.

25. Utilisation selon l'une quelconque des revendications 14 à 24, le procédé comprenant en outre l'incubation desdites cellules dendritiques génétiquement modifiées avec les lymphocytes T auxiliaires CD4+ dudit patient pour obtenir des lymphocytes T stimulés.

26. Utilisation de cellules dendritiques génétiquement modifiées selon la revendication 12, pour la fabrication d'un médicament pour la stimulation de la réponse immunitaire d'un patient souffrant d'une maladie.

27. Composition pharmaceutique comprenant un support pharmaceutiquement acceptable et des cellules dendritiques génétiquement modifiées comprenant un AAV recombinant comprenant au moins un gène hétérologue choisi dans le groupe constitué d'un gène codant un antigène, d'un gène codant une cytokine et de combinaisons d'un ou plusieurs gènes de ceux-ci, intégré de manière stable dans le génome de ladite cellule dendritique.

28. Composition pharmaceutique selon la revendication 27, dans laquelle lesdites cellules dendritiques génétiquement modifiées sont obtenues par un procédé comprenant
(a) la préparation de précurseurs de cellules dendritiques adhérents à partir de précurseurs de cellules dendritiques obtenus auprès d'un sujet humain ; et
(b) la transduction desdits précurseurs de cellules dendritiques adhérents avec au moins un AAV recombinant comprenant au moins un gène hétérologue pour obtenir des cellules dendritiques génétiquement modifiées exprimant ledit gène hétérologue ; et
(c) l'incubation desdits précurseurs de cellules dendritiques adhérents dans un milieu dans des conditions où lesdits précurseurs de cellules dendritiques adhérents se différencient en des cellules dendritiques qui contiennent ledit gène hétérologue intégré de manière stable dans le génome de ladite cellule dendritique pour obtenir des cellules dendritiques génétiquement modifiées.
